(19) Europäisches Patentamt
European Patent Office
Office européen des brevets

(11) **EP 2 431 064 B1**

(12) **EUROPÄISCHE PATENTSCHRIFT**

(45) Veröffentlichungstag und Bekanntmachung des Hinweises auf die Patenterteilung:
**25.03.2015   Patentblatt 2015/13**

(51) Int Cl.:
***A61M 1/36*** *(2006.01)*        *A61M 1/30* *(2006.01)*

(21) Anmeldenummer: **11009771.4**

(22) Anmeldetag: **31.05.2008**

(54) **Vorrichtung zur Steuerung einer Einrichtung zum Fördern von Blut**

Device for controlling a device for conveying blood

Dispositif de commande d'un dispositif de transport de sang

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **04.06.2007   DE 102007026010**

(43) Veröffentlichungstag der Anmeldung:
**21.03.2012   Patentblatt 2012/12**

(62) Dokumentnummer(n) der früheren Anmeldung(en) nach Art. 76 EPÜ:
**08758923.0 / 2 152 336**

(73) Patentinhaber: **Fresenius Medical Care Deutschland GmbH 61352 Bad Homburg v.d.H. (DE)**

(72) Erfinder:
• **Götz, Günther, Dr. 61440 Oberursel (DE)**
• **Herrenbauer, Michael, Dr. 61267 Neu-Anspach (DE)**
• **Lauer, Martin 66606 St. Wendel (DE)**
• **Müller, Ralf, Dr. 61348 Bad Homburg (DE)**

(74) Vertreter: **Oppermann, Frank et al OANDO Oppermann & Oppermann LLP Washingtonstrasse 75 65189 Wiesbaden (DE)**

(56) Entgegenhaltungen:
EP-A- 0 405 094        WO-A-03/072942
US-A- 5 178 603        US-A1- 2001 021 817
US-A1- 2003 152 482

EP 2 431 064 B1

**Beschreibung**

Die Erfindung bezieht sich auf eine Vorrichtung zur Steuerung einer Einrichtung zum Fördern von Blut in einer Blutleitung eines extrakorporalen Blutkreislaufs einer extrakorporalen Blutbehandlungsvorrichtung, wobei es sich bei der extrakorporalen Blutbehandlungsvorrichtung um eine Blutbehandlungsvorrichtung, insbesondere eine Dialysevorrichtung, handeln kann, die im Einnadel-Betrieb oder im Zweinadel-Betrieb betrieben werden kann. Darüber hinaus betrifft die Erfindung eine extrakorporale Blutbehandlungsvorrichtung mit einer derartigen Einrichtung zum Fördern von Blut in einer Blutleitung des extrakorporalen Blutkreislaufs der Blutbehandlungsvorrichtung.

Blutbehandlungsvorrichtungen mit einer Blutbehandlungseinheit, die von dem Blut eines Patienten durchströmt wird, sind allgemein bekannt. Zu diesen zählen beispielsweise die bekannten Hämodialyse-, Hämofiltrations- oder Hämodiafiltrationsvorrichtungen. Die bekannten Blutbehandlungsvorrichtungen können im Einnadel- oder Zweinadel-Betrieb betrieben werden.

Sowohl die Blutbehandlungsvorrichtungen für den Einnadel- als auch den Zweinadel-Betrieb verfügen über einen extrakorporalen Blutkreislauf mit einer arteriellen Blutleitung, die zu der Blutbehandlungseinheit führt, und einer venösen Blutleitung, die von der Blutbehandlungseinheit abgeht.

Bei der Zweinadel-Technik wird das Blut über eine arterielle Nadel von einem Blutgefäß des Patienten abgezogen, in die Blutbehandlungseinheit der Blutbehandlungsvorrichtung geleitet und über eine venöse Nadel in ein Blutgefäß des Patienten zurückgeführt. Für die Entnahme und Rückführung des Blutes finden auswechselbare Schlauchsysteme mit einer Blutzuführ- und -rückführleitung Verwendung, an denen die beiden Nadeln angeschlossen werden. Diese wegzuwerfenden Schlauchsysteme werden auch als Disposable bezeichnet.

Bei der Einnadel-Technik erfolgt die Entnahme und Rückführung des Bluts über eine einzige Nadel, an der sowohl die arterielle als auch die venöse Blutleitung angeschlossen sind. Das dem Patienten entnommene Blut wird während einer arteriellen Phase in einem Reservoir gespeichert, um dann in einer venösen Phase aus dem Speicher in den Blutkreislauf des Patienten durch dieselbe Nadel zurückgeführt zu werden.

Eine Blutbehandlungsvorrichtung für den Einnadel-Betrieb ist aus der EP-A-0 472 480 B 1 bekannt. Bei einer Ausführungsform des bekannten Blutbehandlungsgeräts sind zwei Blutexpansionskammern zum temporären Speichern von Blut vorgesehen, die stromauf und stromab der Blutbehandlungseinheit angeordnet sind. Das Blutbehandlungsgerät weist eine Regeleinrichtung auf, die den Druck in den Expansionskammern im Wesentlichen konstant hält. Zur Detektion der Flüssigkeitspegel in den Expansionskammern sind Niveausensoren vorgesehen.

Die DE 10 2005 001 779 A1 beschreibt ein Set für ein Disposable zum Betreiben einer Blutbehandlungsvorrichtung im Einnadel- oder Zweinadel-Betrieb. Das Disposable umfasst neben der Blutzuführ- und -rückführleitung zum Anschluss an eine Blutbehandlungseinheit eine Expansionseinheit, die für den Einnadel-Betrieb zur Vergrößerung des Volumens an die Luftabscheideeinheit ankoppelbar ist. Während des Einnadel-Betriebs wird in der arteriellen Phase Blut durch die Blutzuführleitung in die Blutbehandlungseinheit und aus der Blutbehandlungseinheit in die Luftabscheide- und Expansionseinheit gefördert, wobei die Blutzufuhr zum Patienten unterbrochen ist. Dabei wird ein vorgegebener Druck in der Luftabscheide- und Expansionseinheit aufgebaut, der mit einer Druckmesseinheit überwacht wird. Mit einer Drucklufteinheit kann durch Betätigung einer Luftpumpe, die zwischen einen Tank und die Expansionseinheit geschaltet ist, ein vorgegebener Druck in der Expansions- und Luftabscheideeinheit eingestellt werden. Darüber hinaus wird vorgeschlagen, mit Hilfe der Messwerte von drei Drucksensoren und der bekannten Systemvolumina das Blutvolumen in der Expansions- und Luftabscheideeinheit zu berechnen. Ferner wird vorgeschlagen, die Luftpumpe zur Regelung des Drucks in der venösen Phase zu verwenden, so dass die Förderrate des Blutes optimal angepasst werden kann.

Für den ordnungsgemäßen Betrieb von Blutbehandlungsvorrichtungen sowohl im Einnadel- als auch im Zweinadel-Betrieb ist von Bedeutung, dass dem Patienten Blut mit einem vorgegebenen Volumenstrom zurückgeführt wird.

Aus der US 5 178 603 A ist ein Verfahren bekannt, bei dem unterhalb eines vorgegebenen Druckgrenzwertes mit einem bestimmten Volumenstrom gefördert wird, wobei nach Erreichen des Druckgrenzwertes mit dem maximal möglichen Volumenstrom gefördert wird. Dem bekannten Verfahren liegt das Prinzip zugrunde, eine maximale Förderrate bei dem maximal möglichen Druck anzustreben.

Aus der US 2003/0152482 A1 ist eine Vorrichtung zur Steuerung des Blutflusses bekannt, die eine Überwachung des Über- und Unterdrucks bei der Entnahme bzw. der Zufuhr von Blut vorsieht. Dabei soll eine maximale Blutflussrate erzielt werden, die einem bestimmten Algorithmus bei der Überwachung des Blutdrucks genügt. Wenn die maximale Flussrate zu einem Überschreiten eines Druckgrenzwertes führt, wird die Flussrate solange reduziert, bis der Druck nicht größer als der Grenzwert ist. Auch die WO 03/072942 A1 schlägt bei Überschreiten eines bestimmten Grenzwertes für den Druck die Reduzierung der Förderrate der Pumpe vor.

Der Erfindung liegt die Aufgabe zu Grunde, eine Vorrichtung zur Steuerung einer Einrichtung zum Fördern von Blut in einer Blutleitung eines extrakorporalen Blutkreislaufs einer extrakorporalen Blutbehandlungsvorrichtung bereitzustellen, die eine optimale Steuerung des Blutflusses unter Vermeidung des Auftretens von Druckspitzen erlaubt.

Darüber hinaus liegt der Erfindung die Aufgabe zugrunde, eine extrakorporale Blutbehandlungsvorrichtung mit einer derartigen Steuerungseinrichtung zu schaffen, bei der eine optimale Steuerung des Blutflusses unter Vermeidung des Auftretens von Druckspitzen erfolgt.

Die Lösung dieser Aufgaben erfolgt erfindungsgemäß mit den Merkmalen der Patentansprüche 1 und 2. Vorteilhafte Ausführungsformen der Erfindung sind Gegenstand der Unteransprüche.

Die erfindungsgemäße Vorrichtung beruht darauf, dass ein bestimmter Grenzwert für den Druck in der Blutleitung vorgegeben wird, der nicht überschritten werden sollte. Der Grenzwert für den maximalen Druck in der Blutleitung kann dabei so bemessen sein, dass der Grenzwert über dem Druck liegt, bei dem die Alarmeinrichtungen ansprechen, die im Allgemeinen in den bekannten extrakorporalen Blutbehandlungsvorrichtungen vorgesehen sind. Im Allgemeinen wird als Grenzwert für den Druck nicht ein absoluter Wert für den Druck angenommen, sondern ein relativer Wert bezogen auf den Umgebungsdruck. Eine Änderung des Umgebungsdrucks führt somit nicht zu einer Änderung des Grenzwertes relativ zu diesem Druck. Grundsätzlich kann der Grenzwert aber auch ein absoluter Wert für den Druck sein.

Bei der extrakorporalen Blutbehandlungsvorrichtung können grundsätzlich zwei Grenzwertfenster für den Druck vorgegeben werden, wobei sich das eine Grenzwertfenster auf den Überdruck in der venösen Blutleitung bezieht, mit dem das Blut dem Patienten zugeführt wird, und das andere Grenzwertfenster sich auf den Unterdruck in der arteriellen Blutleitung bezieht, mit dem das Blut dem Patienten entnommen wird. Durch die Überwachung beider Grenzwertfenster wird sichergestellt, dass der Blutfluss in der extrakorporalen Blutbehandlungsvorrichtung ordnungsgemäß ist.

Darüber hinaus beruht die erfindungsgemäße Vorrichtung darauf, dass die Einrichtung zum Fördern von Blut derart angesteuert wird, dass das Blut in der Blutleitung mit einem vorgegebenen Volumenstrom so lange gefördert wird, wie der Druck in der Blutleitung unterhalb des vorgegebenen Grenzwertes liegt. Wenn der Druck in der Blutleitung jedoch den vorgegebenen Grenzwert erreicht, wird die Einrichtung zum Fördern von Blut derart angesteuert, dass sich beim Fördern des Bluts in der Blutleitung ein Druck einstellt, der dem Grenzwert entspricht. Folglich wird bei Erreichen des Grenzwertes für den Druck von einer Regelung des Volumenstroms auf eine Regelung des Drucks in der Blutleitung übergegangen, ohne dass die Einrichtung zum Fördern von Blut sofort angehalten wird.

Solange der Druck in der Blutleitung nicht den vorgegebenen Grenzwert erreicht, führen weder kurzfristige Änderungen des Flusswiderstandes, z.B. infolge von Änderungen der Lage der Nadel, beispielsweise durch Bewegen des Arms des Patienten, noch langsame Änderungen des Flusswiderstandes, z.B. beim Anstieg des Hämatokrits, während der Blutbehandlung zu Änderungen des Volumenstroms des Bluts.

Da der Druck, mit dem Blut dem Patienten zugeführt wird (Überdruck) oder der Druck, mit dem Blut dem Patienten entnommen wird (Unterdruck) auf einen vorgegebenen Wert begrenzt wird, werden unerwünschte Druckspitzen im extrakorporalen Blutkreislauf vermieden, die das Blutgefäßsystem (Shunt) des Patienten schädigen könnten.

Die erfindungsgemäße Steuerungseinrichtung ist grundsätzlich Bestandteil einer extrakorporalen Blutbehandlungsvorrichtung, die über eine Einrichtung zum Fördern von Blut in einer Blutleitung eines extrakorporalen Blutkreislaufs verfügt. Dabei kann die erfindungsgemäße Steuerungseinrichtung von den in den bekannten Blutbehandlungsvorrichtungen im Allgemeinen bereits vorhandenen Komponenten Gebrauch machen.

Die extrakorporale Blutbehandlungsvorrichtung mit der erfindungsgemäßen Steuerungseinrichtung kann eine Blutbehandlungsvorrichtung für den Betrieb mit zwei Patientenkonnektionsstellen (Zweinadel-Betrieb) sein, bei der die arterielle Blutleitung eine arterielle Patientenkonnektionsstelle und die venöse Blutleitung eine venöse Patientenkonnektionsstelle aufweist. Bei einer derartigen Blutbehandlungsvorrichtung ist die Einrichtung zum Fördern von Blut eine Blutpumpe, die im extrakorporalen Blutkreislauf, insbesondere in der arteriellen Blutleitung angeordnet ist.

Bei einer extrakorporalen Blutbehandlungsvorrichtung für den Zweinadel-Betrieb kann ein Wert für den Betrag des maximalen Drucks in der venösen Blutleitung und/oder der arteriellen Blutleitung vorgegeben werden, um sicherzustellen, dass nicht mit einem zu hohen Überdruck Blut dem Patienten zurückgeführt und/oder mit einem zu hohen Unterdruck dem Patienten Blut entnommen wird.

Die entscheidenden Vorteile der erfindungsgemäßen Steuerungseinrichtung kommen insbesondere bei einer extrakorporalen Blutbehandlungsvorrichtung für den Betrieb mit einer Patientenkonnektionsstelle (Einnadel-Betrieb) zum Tragen, bei der die arterielle Blutleitung und die venöse Blutleitung eine gemeinsame Patientenkonnektionsstelle aufweisen.

Die Blutbehandlungsvorrichtung für den Einnadel-Betrieb weist in der venösen Blutleitung des extrakorporalen Blutkreislaufs angeordnete Mittel zum Sammeln von Blut auf, die ein vorgegebenes Volumen umfassen. Die Mittel zum Sammeln von Blut können aber auch in der arteriellen Leitung angeordnet sein. Die Einrichtung zum Fördern von Blut ist bei der Blutbehandlungsvorrichtung für den Einnadel-Betrieb eine Einrichtung zum Erzeugen eines vorgegebenen Drucks in den Mitteln zum Sammeln von Blut, so dass in den Mitteln zum Sammeln von Blut gesammeltes Blut aus den Mitteln zum Sammeln von Blut verdrängt wird.

Die Einrichtung zum Erzeugen eines vorgegebenen Drucks in den Mitteln zum Sammeln von Blut kann unterschiedlich ausgebildet sein. Beispielsweise kann die Einrichtung zum Erzeugen eines vorgegebenen Drucks ein Kompressor sein, mit dem ein Überdruck erzeugt werden kann.

Bei der extrakorporalen Blutbehandlungsvorrichtung für den Einnadel-Betrieb wird während einer arteriellen Phase Blut zu den Mitteln zum Sammeln von Blut, die ein abgeschlossenes Volumen umfassen, gefördert, und während einer venösen Phase in den Mitteln zum Sammeln von Blut ein vorgegebener Druck aufgebaut, so dass in den Mitteln zum Sammeln von Blut gesammeltes Blut verdrängt wird. Dabei wird fortlaufend zwischen der arteriellen und der venösen Phase umgeschaltet.

Bei der extrakorporalen Blutbehandlungsvorrichtung für den Einnadel-Betrieb kann vom Benutzer ein Grenzwert für den Druck in der venösen Blutleitung, d.h. der maximale Rückgabedruck, und ein bestimmter Volumenstrom, d.h. ein Soll-Volumenstrom, vorgegeben werden. Der maximale Rückgabedruck ist vorzugsweise ein relativer Druck bezogen auf den Umgebungsdruck. Er kann grundsätzlich aber auch ein absoluter Druck sein.

Im Gegensatz zu einem Verfahren, bei dem in dem abgeschlossenen Volumen der Mittel zum Sammeln von Blut ein vorgegebener Soll-Druck eingestellt wird, der während der venösen Phase aufrechterhalten werden soll, wird bei der erfindungsgemäßen Steuerungseinrichtung bzw. dem erfindungsgemäßen Verfahren in dem abgeschlossenen Volumen der Mittel zum Sammeln von Blut der Druck derart geregelt, dass sich in der venösen Blutleitung der vorgegebene Volumenstrom ergibt. Hierzu wird der vom Benutzer vorgegebene Soll-Volumenstrom mit dem gemessenen oder berechneten Ist-Volumenstrom verglichen. Folglich strömt das Blut in der venösen Blutleitung mit dem vom Benutzer vorgegebenen Soll-Volumenstrom, solange der Rückgabedruck nicht den vom Benutzer vorgegebenen Maximaldruck überschreitet.

Wenn sich der Flusswiderstand kurzzeitig ändert, z.B. bei einer Änderung der Lage der Patientenkonnektionsstelle, kann der Rückgabedruck bis zu dem vorgegebenen Maximaldruck erhöht werden, so dass der effektive Volumenstrom konstant gehalten wird, solange der maximale Rückgabedruck nicht überschritten wird. Auch langsame Änderungen des Flusswiderstandes, beispielsweise bei einem Anstieg des Hämatokrits führen zu einer Erhöhung des Rückgabedrucks bis zum maximalen Rückgabedruck, so dass der Volumenstrom konstant gehalten wird, solange der maximale Rückgabedruck nicht überschritten wird.

Eine bevorzugte Ausführungsform der Erfindung sieht vor, dass die Regelung des Volumenstroms mit einem ersten Regelkreis erfolgt, der Mittel zum Berechnen eines Soll-Drucks für das abgeschlossene Volumen der Mittel zum Sammeln von Blut aufweist, der derart bemessen ist, dass sich in der venösen Blutleitung der vorgegebene Volumenstrom einstellt, und mit einem zweiten Regelkreis erfolgt, der die Einrichtung zur Erzeugung eines vorgegebenen Drucks in den Mitteln zum Sammeln von Blut derart ansteuert, dass sich der berechnete Soll-Druck in dem abgeschlossenen Volumen der Mittel zum Sammeln von Blut auch einstellt.

Eine weitere besonders bevorzugte Ausführungsform sieht vor, dass bei der extrakorporalen Blutbehandlungsvorrichtung für den Einnadel-Betrieb Luft von Mitteln zum Speichern von Luft zu den Mitteln zum Sammeln von Blut mit Mitteln zum Verdichten von Luft überführt wird, die in einem Verbindungspfad zwischen den Mitteln zum Sammeln von Blut und den Mitteln zum Speichern von Luft angeordnet sind. Bei dieser Ausführungsform bilden die Mittel zum Sammeln von Blut und die Mittel zum Speichern von Luft zusammen mit dem Verbindungspfad ein abgeschlossenes Volumen, in das grundsätzlich keine Luft eintreten bzw. aus dem keine Luft austreten kann.

Im Folgenden werden verschiedene Ausführungsbeispiele der Erfindung unter Bezugnahme auf die Zeichnungen im Einzelnen erläutert.

Es zeigen:

Fig. 1 Die wesentlichen Komponenten einer extrakorporalen Blutbehandlungsvorrichtung für den Zweinadel-Betrieb in stark vereinfachter schematischer Darstellung, die über eine Vorrichtung zur Steuerung einer Einrichtung zum Fördern von Blut verfügt,

Fig. 2 ein Wirkplan zur Veranschaulichung des Funktionsprinzips der Blutbehandlungsvorrichtung von Figur 1,

Fig. 3 eine extrakorporale Blutbehandlungsvorrichtung für den Einnadel-Betrieb mit der erfindungsgemäßen Steuerungseinrichtung in stark vereinfachter schematischer Darstellung,

Fig. 4 den Verlauf des Füllstandes und des Drucks während der Initialisierung und des Betriebs der Blutbehandlungsvorrichtung von Fig. 3,

Fig. 5 den Druckverlauf während der aufeinander folgenden arteriellen und venösen Phasen während des Betriebs der Blutbehandlungsvorrichtung von Fig. 3,

Fig. 6 ein Blockschaltbild der erfindungsgemäßen Steuerungseinrichtung der erfindungsgemäßen Blutbehandlungsvorrichtung von Fig. 3 und

Fig. 7 ein Blockschaltbild des Druckreglers der Steuerungseinrichtung von Fig. 6.

Die erfindungsgemäße Steuerungseinrichtung kann sowohl bei einer extrakorporalen Blutbehandlungsvorrichtung für den Zweinadel-Betrieb als auch für den Einnadel-Betrieb Verwendung finden. Zunächst wird eine extrakorporale Blutbehandlungsvorrichtung für den Zweinadel-Betrieb mit der Steuerungseinrichtung beschrieben.

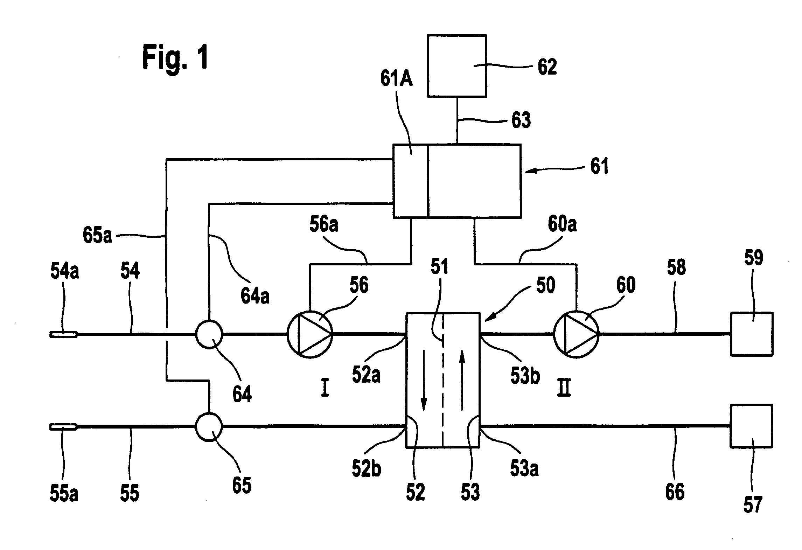

Fig. 1 zeigt die wesentlichen Komponenten einer extrakorporalen Blutbehandlungsvorrichtung für den Zweinadel-Betrieb, insbesondere Hämodialysevorrichtung, in schematischer Darstellung. Die Anordnung weist einen Dialysator 50 auf, der durch eine semipermeable Membran 51 in eine Blutkammer 52 und eine Dialysierflüssigkeitskammer 53 getrennt ist.

Eine arterielle Blutleitung 54 mit einer arteriellen Patientenkonnektionsstelle 54a führt zu einem Einlass 52a der Blutkammer 52, während von einem Auslass 52b der Blutkammer 52 des Dialysators 50 eine venöse Blutleitung 55 mit einer venösen Patientenkonnektionsstelle 55a abgeht. Bei den Patientenkonnektionsstellen kann es sich um Kanülen oder Nadeln, aber auch um Konnektionselemente handeln, mit denen der extrakorporale Kreislauf an bereits bestehende Patientenverbindungen wie verlegte Katheter angeschlossen wird. In der arteriellen Blutleitung 54 ist eine Blutpumpe 56, insbesondere Rollenpumpe, angeordnet, die das Blut im extrakorporalen Blutkreislauf I fördert. Die arterielle und venöse Blutleitung 54, 55 sind als Disposable ausgebildet, das in die Hämodialysevorrichtung eingelegt wird. Auch der Dialysator 50 sowie die Patientenkonnektionsstellen 55a und 55b sind nur zur einmaligen Verwendung bestimmt. Anstelle einer Rollenpumpe kann grundsätzlich aber auch eine Blutpumpe anderer Bauart Verwendung finden.

Der Dialysierflüssigkeitskreislauf II umfasst eine Dialysierflüssigkeitszuführleitung 56, die von einer Dialysierflüssigkeitsquelle 57 abgeht und zu einem Einlass 53a der Dialysierflüssigkeitskammer 53 führt, und eine Dialysierflüssigkeitsrückführleitung 58, die von einem Auslass 53b der Dialysierflüssigkeitskammer 53 des Dialysators 50 abgeht und zu einem Auslass 59 führt. In der Dialysierflüssigkeitsrückführleitung 58 ist eine Dialysierflüssigkeitspumpe 60 zum Fördern der Dialysierflüssigkeit im Dialysierflüssigkeitskreislauf II angeordnet. Dem Fachmann sind zum Fördern der Dialysierflüssigkeit unterschiedlichste Pumpenarten und Bilanziereinrichtungen geläufig, ohne dass an dieser Stelle hierauf eingegangen zu werden braucht.

Darüber hinaus verfügt die Hämodialysevorrichtung über eine zentrale Rechen- und Steuereinheit 61 und eine Eingabeeinheit 62, die über eine Datenleitung 63 miteinander verbunden sind. Die zentrale Steuer- und Recheneinheit 61, die über eine Steuerleitung 56a mit der Blutpumpe 56 und eine Steuerleitung 60a mit der Dialysierflüssigkeitspumpe 60 verbunden ist, steuert die Blutpumpe und die Dialysierflüssigkeitspumpe an, so dass beide Pumpen mit einer bestimmten Drehzahl betrieben werden.

Weiterhin ist ein arterieller Drucksensor 64 zum Messen des Drucks in der arteriellen Blutleitung 54 stromauf der Blutpumpe 56 und ein venöser Drucksensor 65 zum Messen des Drucks in der venösen Blutleitung 55 vorgesehen. Die Messwerte beider Drucksensoren 64, 65 werden mit Datenleitungen 64a, 65a an die zentrale Steuer- und Recheneinheit 61 übertragen.

Vom Benutzer kann ein bestimmter Volumenstrom für das in der venösen Blutleitung 55 strömende Blut vorgegeben werden. Dieser Volumenstrom kann mit der Eingabeeinheit 62, beispielsweise mit einer Tastatur, eingegeben werden. Es ist aber auch möglich, dass ein bestimmter Wert für den Volumenstrom von der Dialysevorrichtung vorgegeben wird.

Des Weiteren kann der Benutzer einen maximal tolerierbaren Rückgabedruck in der venösen Blutleitung 55, mit dem das Blut dem Patienten zurückgeführt wird, und einen maximal tolerierbaren - den Betrag des Unterdrucks betreffend - Saugdruck in der arteriellen Blutleitung 54 stromauf der Blutpumpe 56 vorgeben, mit dem das Blut dem Patienten entnommen wird. Die Druckwerte kann der Benutzer mit der Eingabeeinheit 62 eingeben. Es ist aber auch möglich, dass die Dialysevorrichtung Werte für den maximal tolerierbaren Rückgabedruck bzw. den minimalen Saugdruck vorgibt. Vorzugsweise ist der Rückgabe- bzw. Saugdruck ein relativer Druck bezogen auf den Umgebungsdruck, wobei hier der Rückgabedruck mit einem positiven Wert und der Saugdruck mit einem negativen Wert angegeben wird.

Die Hämodialysevorrichtung verfügt über eine Einrichtung zur Steuerung der Blutpumpe 61A, mit der das Blut in der arteriellen und venösen Blutleitung gefördert wird. Die Steuerungseinrichtung 61A ist bei dem vorliegenden Ausführungsbeispiel Bestandteil der zentralen Rechen- und Steuereinheit 61, sie kann aber auch eine separate Einheit bilden. Die Steuerungseinrichtung 61A erzeugt für die Blutpumpe 56 ein Steuersignal, mit dem die Blutpumpe angesteuert wird, wobei die Drehzahl der Blutpumpe in Abhängigkeit von dem Steuersignal eingestellt wird.

Die Steuerungseinrichtung 61A vergleicht den gemessenen venösen Überdruck in der venösen Blutleitung 55 stromab der Blutpumpe 56, der vorzugsweise ein bezogen auf den Umgebungsdruck relativer Druck ist, mit dem vorgegebenen maximal tolerierbaren Rückgabedruck und vergleicht den gemessenen arteriellen Unterdruck in der arteriellen Blutleitung 54 stromauf der Blutpumpe 56, der ebenfalls vorzugsweise ein bezogen auf den Umgebungsdruck relativer Druck ist, mit dem vorgegebenen minimal tolerierbaren Saugdruck.

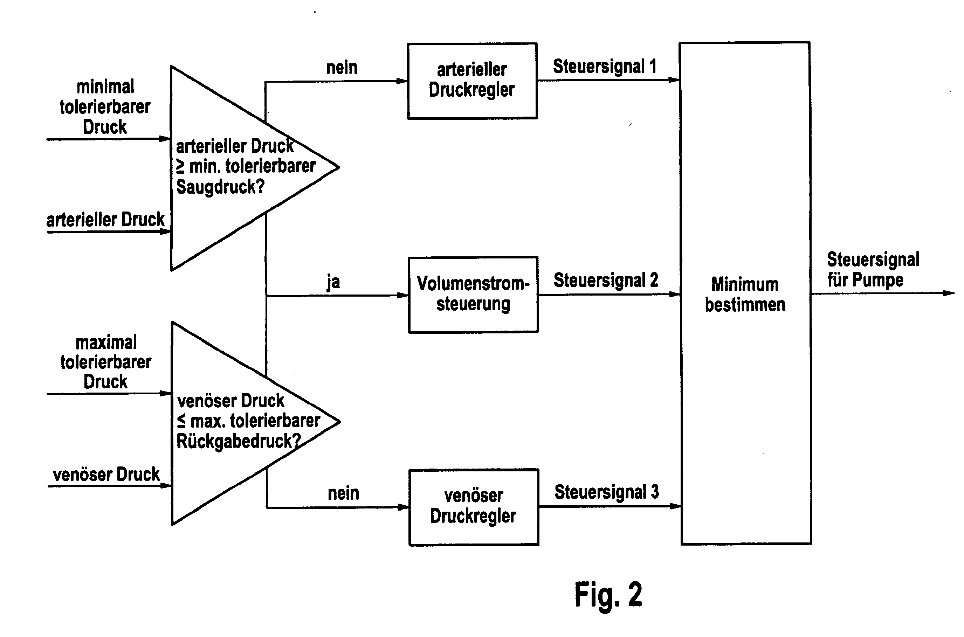

Für den Fall, dass sowohl der Wert des venösen Rückgabedrucks kleiner oder gleich als der maximal tolerierbare Rückgabedruck ist als auch der Wert des arteriellen Saugdrucks größer oder gleich als der minimal tolerierbare Rückgabedruck ist, sieht die Steuerungseinrichtung eine Volumensteuerung mit einem Steuersignal 2 für die Blutpumpe vor (Fig. 2).

Für den Fall jedoch, dass entweder der arterielle Druck (Unterdruck) kleiner als der minimal tolerierbare Saugdruck oder der venösen Druck (Überdruck) größer als der maximal tolerierbare Rückgabedruck ist, sieht die Steuereinrichtung von der Volumensteuerung ab. In diesem Fall erfolgt eine arterielle oder venöse Druckregelung.

Für die arterielle Druckregelung verfügt die Steuerungseinrichtung 61 A über einen arteriellen Druckregler und für die venöse Druckregelung über einen venösen Druckregler. Der arterielle Druckregler erzeugt ein Steuersignal 1 für die Blutpumpe, das derart bemessen ist, dass sich in der arteriellen Blutleitung 54 stromauf der Blutpumpe 56 der minimal tolerierbare Saugdruck einstellt, d.h. die Drehzahl der Blutpumpe wird so geregelt, dass der Druck konstant gehalten wird, während der venöse Druckregler ein Steuersignal 3 erzeugt, das derart bemessen ist, dass sich in der venösen Blutleitung 55 der maximal tolerierbare Rückgabedruck einstellt, d.h. die Drehzahl der Blutpumpe wird so geregelt, dass der Druck in der venösen Blutleitung konstant gehalten wird.

Darüber hinaus verfügt die Steuerungseinrichtung 61A über eine Einrichtung zur Auswahl eines der drei Steuersignale 1, 2, 3 für den Betrieb der Blutpumpe 56. Im Normalbetrieb erfolgt eine Volumenstromsteuerung mit dem Steuersignal 2. Ansonsten wählt die Auswahleinrichtung aus den beiden Steuersignalen 1 bzw. 3 das Steuersignal für die Blutpumpe aus, das dem "Minimum" der Steuersignale 1 bzw. 3 entspricht, d.h. die Blutpumpe wird derart mit einem Steuersignal betrieben, dass keines der beiden zulässigen Grenzwerte über- bzw. unterschritten wird.

Wenn also der Wert des arteriellen Drucks kleiner als der minimal tolerierbare Saugdruck ist, aber der Wert des venösen Drucks noch kleiner als der maximale Rückgabedruck ist, wird die Blutpumpe mit dem Steuersignal 1 derart angesteuert, dass dem Patienten Blut mit dem maximal tolerierbaren Saugdruck entnommen wird. Um den Saugdruck konstant zu halten, wird die Drehzahl der Blutpumpe kontinuierlich verändert. Wenn der Wert des venösen Drucks größer als der maximal tolerierbare Rückgabedruck ist, der Wert des arteriellen Drucks aber noch größer als der minimale Saugdruck ist, wird die Blutpumpe mit dem Steuersignal 3 angesteuert, so dass deren Drehzahl in Abhängigkeit von dem venösen Druck geregelt wird, dass dem Patienten Blut mit dem maximal tolerierbaren Rückgabedruck zugeführt wird. Wenn jedoch sowohl der arterielle Druck kleiner als der minimale Saugdruck und der venöse Druck größer als der maximale Rückgabedruck ist, wird die Blutpumpe so betreiben, dass weder der minimale Saugdruck unterschritten noch der maximale Rückgabedruck überschritten wird. Die Blutpumpe wird also derart betrieben, dass sich entweder der minimale Saugdruck oder der maximale Rückgabedruck in der Blutleitung einstellt, wobei die Auswahl so getroffen wird, dass keiner der beiden Werte außerhalb des Grenzwertfensters liegt.

Die erfindungsgemäße Steuerungseinrichtung kann auch bei Blutbehandlungsvorrichtungen für den Einnadel-Betrieb Verwendung finden. Wenn die Blutbehandlungsvorrichtung für den Einnadel-Betrieb über zwei Blutpumpen verfügt, mit denen das Blut im arteriellen und venösen Zweig des extrakorporalen Blutkreislaufs gefördert wird, können mit der erfindungsgemäßen Steuerungseinrichtung beide Blutpumpen nach dem erfindungsgemäßen Verfahren angesteuert werden.

Die Vorteile der erfindungsgemäßen Steuerungseinrichtung und des erfindungsgemäßen Steuerungsverfahrens zeigen sich insbesondere bei einer Blutbehandlungsvorrichtung für den Einnadel-Betrieb, bei der das dem Patienten entnommene Blut in einer arteriellen Phase in Mitteln zum Sammeln von Blut gesammelt wird und in einer venösen Phase aus den Mitteln zum Sammeln von Blut dem Patienten wieder zugeführt wird, indem die Mittel zum Sammeln von Blut mit Druck beaufschlagt werden, so dass das in den Mitteln zum Sammeln von Blut gesammelte Blut verdrängt wird. Nachfolgend wird eine derartige bevorzugte Ausführungsform der Blutbehandlungsvorrichtung im Einzelnen erläutert.

Zunächst wird der Aufbau und die Funktionsweise der Blutbehandlungsvorrichtung für den Einnadel-Betrieb als solche beschrieben, bevor Aufbau und Funktionsweise der Steuerungseinrichtung beschrieben wird, die bei dem vorliegenden Ausführungsbeispiel Bestandteil der Blutbehandlungsvorrichtung ist.

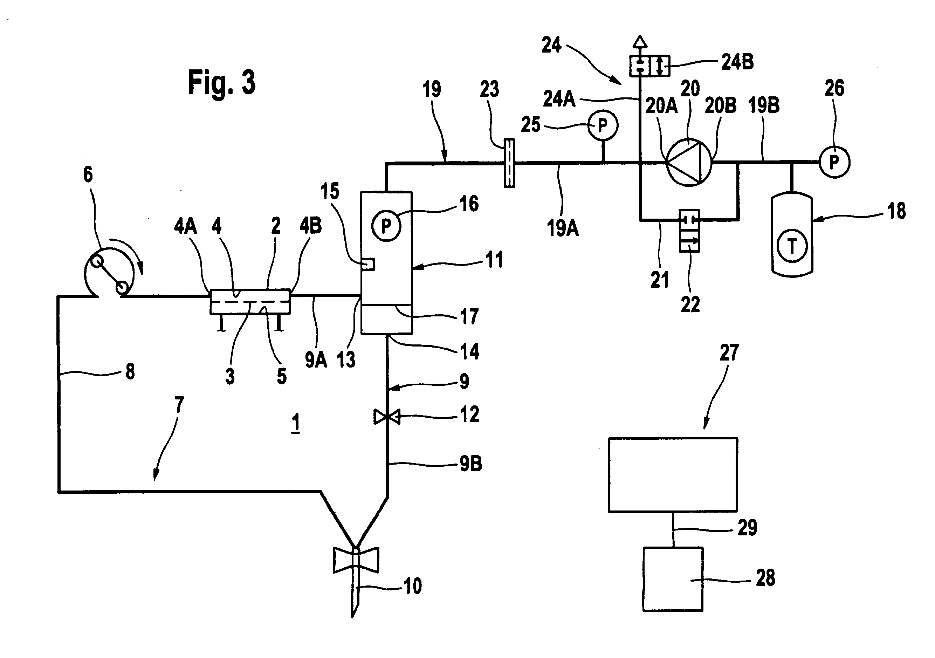

Fig. 3 zeigt die wesentlichen Komponenten der Blutbehandlungsvorrichtung für den Einnadel-Betrieb, insbesondere einer Dialysevorrichtung, in schematischer Darstellung.

Während der Blutbehandlung verfügt die Dialysevorrichtung über einen extrakorporalen Blutkreislauf 1, der eine Blutbehandlungseinheit 2, beispielsweise einen als Disposable ausgebildeten Dialysator umfasst. Der Dialysator 2 ist durch eine semipermeable Membran 3 in eine Blutkammer 4 und eine Dialysierflüssigkeitskammer 5 unterteilt.

Im extrakorporalen Blutkreislauf wird das Blut mittels einer Blutpumpe 6, insbesondere eine Rollenpumpe, oder aber auch mittels einer Pumpe anderer Bauart gefördert, die Teil der Dialysevorrichtung ist. Der in Fig. 3 nicht dargestellte Dialysierflüssigkeitskreislauf kann so ausgebildet sein, wie unter Bezugnahme auf Fig. 1 beschrieben ist.

In die Dialysevorrichtung wird ein Schlauchset 7 eingelegt, das nach der Behandlung verworfen wird. Das Disposable 7 weist eine zu dem Einlass 4A der Blutkammer 4 des Dialysators 2 führende Blutzuführleitung 8, die in die Rollenpumpe 6 der Dialysevorrichtung eingelegt ist, und eine von dem Auslass 4B der Blutkammer abgehende Blutrückführleitung 9 auf. Blutzuführ- und -rückführleitung 8, 9 sind an einer gemeinsamen Patientenkonnektionsstelle 10 (Kanüle oder Nadel) angeschlossen. In der Blutrückführleitung 9 sind Mittel 11 zum Sammeln von Blut angeordnet, die als ein Behälter mit einem vorgegebenen Volumen ausgebildet sind. Nachfolgend werden die Mittel zum Sammeln von Blut als Blutsammelbehälter oder Blutspeicher bezeichnet.

Stromab des Blutsammelbehälters 11 sind an der Blutrückführleitung 9 Mittel 12 zum Unterbrechen der Blutrückführleitung, beispielsweise eine von der Dialysevorrichtung betätigbare venöse Schlauchklemme angeordnet.

Der Blutsammelbehälter 11 weist einen Einlass 13 auf, zu dem ein erster Abschnitt 9A der Blutrückführleitung 9 führt, und weist einen Auslass 14 auf, von dem ein zweiter Abschnitt 9B der Blutrückführleitung 9 abgeht. Zur Detektion eines bestimmten Füllstandes im Blutsammelbehälter 11 weist die Dialysevorrichtung einen Füllstandgeber 15 auf, der detektiert, wenn der Füllstand in dem Behälter einen vorgegebenen Wert erreicht. Darüber hinaus ist ein Druckgeber 16 vorgesehen, der den Druck im Blutsammelbehälter 11 misst.

Wenn der Blutsammelbehälter 11 mit Blut befüllt ist, verbleibt oberhalb des Flüssigkeitsspiegels 17 ein bestimmtes Luftvolumen in dem Blutspeicher. Der Blutsammelbehälter steht in Strömungsverbindung mit Mitteln 18 zum Speichern von Gas, insbesondere Luft, die als ein Behälter mit einem abgeschlossenen Volumen ausgebildet sind. Nachfolgend werden die Mittel 18 zum Speichern von Gas als Luftspeicherbehälter oder Luftspeicher bezeichnet.

Damit Blutspeicher und Luftspeicher miteinander kommunizieren können, geht von der Oberseite des Blutspeichers 11 eine Leitung 19 ab, die zu dem Luftspeicher 18 führt. In der Leitung 19 sind Mittel 20 zum Verdichten von Gas angeordnet, die beispielsweise als konventioneller Kompressor ausgebildet sein können. Solange der Kompressor nicht betrieben wird, unterbricht der Kompressor die Strömungsverbindung zwischen Blutspeicher und Luftspeicher. Beim Betrieb des Kompressors wird hingegen in dem Luftspeicher befindliche Luft in den Blutspeicher überführt. Da die Luft komprimiert wird, baut sich in dem Blutspeicher ein vorgegebener Druck auf.

Die Leitung 19 weist zwei Leitungsabschnitte 19A, 19B auf, von denen der eine Leitungsabschnitt 19A den Blutspeicher mit dem druckseitigen Anschluss 20A des Kompressors 20 und der andere Leitungsabschnitt 19B den saugseitigen Anschluss 20B des Kompressors 20 mit dem Luftspeicher 18 verbindet. Diese Leitungsabschnitte 19A, 19B bilden einen Verbindungspfad zum Überführen von Gas aus dem Luftspeicher in den Blutspeicher.

Um bei nicht im Betrieb befindlichem Kompressor Luft aus dem Blutspeicher in den Luftspeicher überführen zu können, ist eine Bypassleitung 21 vorgesehen, die von dem ersten Leitungsabschnitt 19A der Leitung 19 abgeht und zu dem zweiten Leitungsabschnitt 19B der Leitung 19 führt. In die Bypassleitung 21 ist ein Bypassventil 22 geschaltet. Zusammen mit den entsprechenden Leitungsabschnitten der Leitung 19 bildet die Bypassleitung 21 einen Verbindungspfad zum Fördern von Gas aus dem Blutspeicher in den Luftspeicher.

Um zu verhindern, dass Flüssigkeit aus dem Blutspeicher in den Luftspeicher gelangen kann, ist in dem ersten Leitungsabschnitt 19A der Leitung 19 ein Filter 23 angeordnet, der eine hydrophobe, d.h. für Luft durchlässige, aber für Flüssigkeit undurchlässige Membran enthält. Da der Blutspeicher nur bis zu einem maximalen Füllstand befüllt wird, kann aber ohnehin nur im Falle einer Störung Flüssigkeit in die Leitung 19 gelangen.

Zum Be- / Entlüften des abgeschlossenen Volumens, das den Blutspeicher und den Luftspeicher sowie die Leitung 19 umfasst, sind Mittel 24 zum Be- / Entlüften vorgesehen, die eine an den ersten Leitungsabschnitt 19A der Leitung 19 angeschlossene Be- / Entlüftungsleitung 24A mit einem Be- / Entlüftungsventil 24B aufweisen. Die Be-/Entlüftungsleitung 24A kann grundsätzlich von jeder Stelle des zu be- oder entlüftenden Volumens abgehen. Insbesondere sollte die Be- /entlüftung im maschinenseitigen Teil erfolgen.

Neben dem Druckgeber 16 zum Messen des Drucks in dem Blutspeicher ist ein Druckgeber 25 zum Messen des Drucks in dem ersten Leitungsabschnitt 19A der Leitung 19 zwischen dem Filter 23 und dem Kompressor 20 und ein weiterer Druckgeber 26 zum Messen des Drucks im Luftspeicher 18 vorgesehen. Am Luftspeicher ist ein Temperatursensor T zur Messung der Temperatur der im Luftspeicher befindlichen Luft vorgesehen.

Die Dialysevorrichtung verfügt über eine zentrale Steuer- und Recheneinheit 27, die über nicht dargestellte elektrische Leitungen mit der Blutpumpe 6, der venösen Schlauchklemme 12, dem Bypassventil 22, dem Be- / Entlüftungsventil 24B, dem Füllstandsgeber 15, den Kompressor 20 sowie den Druckgebern 16, 25 und 26 verbunden ist.

Darüber hinaus verfügt die Dialysevorrichtung über eine Eingabeeinheit 28, die mit der Steuer- und Recheneinheit 27 über eine Datenleitung 29 verbunden ist. In die Eingabeeinheit, die beispielsweise als Tastatur ausgebildet ist, kann der Benutzer einen Volumenstrom für das Blut in der venösen Blutleitung 9 und einen maximal zulässigen Wert für den Rückgabedruck in der venösen Blutleitung 9 eingeben. Der Volumenstrom und/oder Rückgabedruck kann aber auch von der Dialysevorrichtung vorgegeben werden.

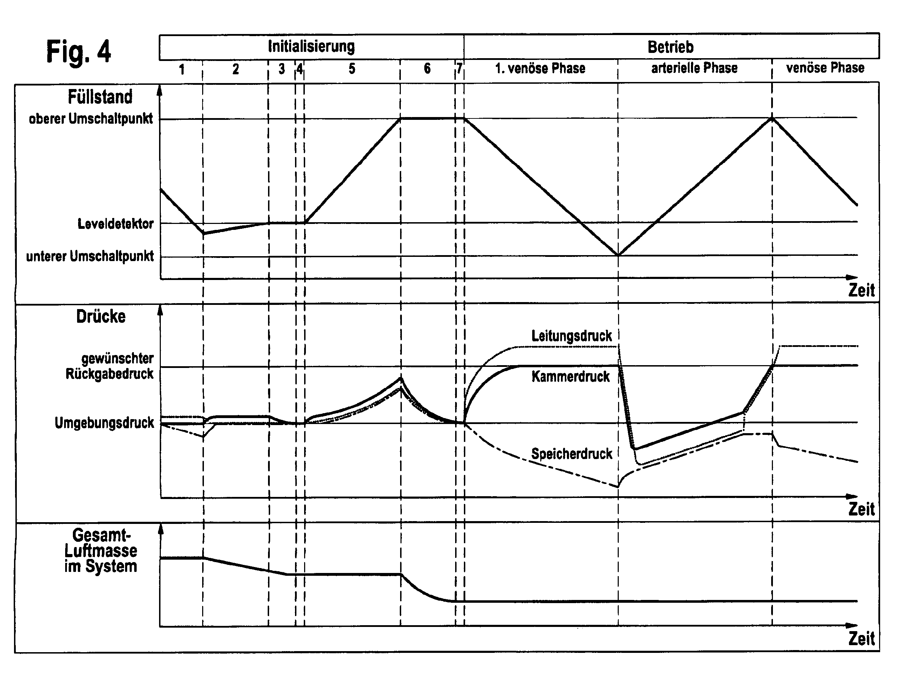

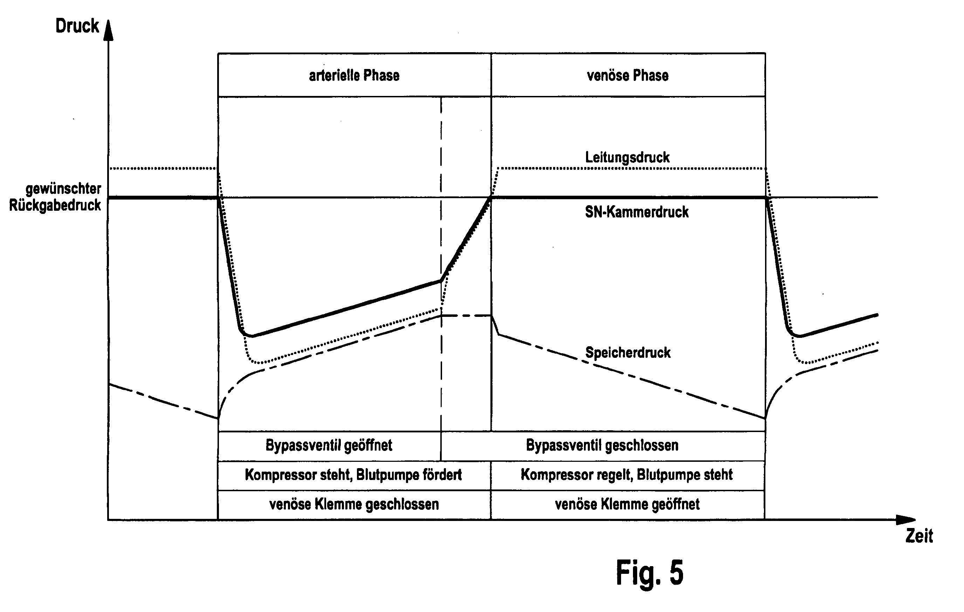

Nachfolgend wird der Betrieb der Dialysevorrichtung unter Bezugnahme auf die Figuren 4 und 5 im Einzelnen beschrieben. Die zentrale Steuer- und Recheneinheit 27 steuert die Dialysemaschine wie folgt.

Zu Beginn der eigentlichen Dialysebehandlung wird eine Initialisierung des Systems mit den folgenden Verfahrensschritten durchgeführt.

Fig. 4 zeigt den Füllstand in dem Blutspeicher als Funktion der Zeit während der einzelnen Phasen der Initialisierung. Darüber hinaus zeigt Fig. 4 den Verlauf des Drucks in dem Blutspeicher, der als Kammerdruck bezeichnet wird, des Drucks in dem Luftspeicher, der als Speicherdruck bezeichnet wird, und des Drucks in dem ersten Leitungsabschnitt 19A der Leitung 19, der als Leitungsdruck bezeichnet wird. Des Weiteren zeigt Fig. 4 den zeitlichen Verlauf der in dem Blutspeicher und im Luftspeicher sowie den entsprechenden Abschnitten der Leitungen 19, 22 eingeschlossenen Gesamt-Luftmasse.

In dem ersten Initialisierungsschritt wird der Blutpegel in dem Blutspeicher unterhalb eines bestimmten Niveaus abgesenkt, das zwischen dem oberen Umschaltpunkt, bei dem während des Betriebs der Dialysevorrichtung von der arteriellen Phase auf die venöse Phase umgeschaltet werden soll und dem unteren Umschaltpunkt liegt, bei dem von der venösen auf die arterielle Phase umgeschaltet werden soll. Hierzu öffnet die Steuer- und Recheneinheit 27 bei still stehender Blutpumpe 6 die venöse Schlauchklemme 12 und setzt den Kompressor 20 so lange in Betrieb, bis das gewünschte Flüssigkeitsniveau erreicht ist, das der Füllstandgeber detektiert. Fig. 4 zeigt, dass der Füllstand absinkt, während die Gesamt-Luftmasse im System konstant bleibt. Dieser Schritt kann übersprungen werden, wenn sich der Blutpegel bereits unterhalb des gewünschten Niveaus befindet.

In dem zweiten Schritt wird der Blutpegel dann auf das gewünschte Niveau eingestellt, das der Füllstandsgeber detektiert. Hierzu wird das Bypassventil 22 und das Be- / Entlüftungsventil 24B geöffnet und die Blutpumpe 6 so lange bei geschlossener venöser Schlauchklemme betrieben, bis das gewünschte Niveau erreicht ist. Fig. 4 zeigt, dass der Füllstand auf das gewünschte Niveau ansteigt, während die Luftmasse im System abnimmt.

Wenn das gewünschte Niveau erreicht ist, wird so lange gewartet, bis sich der Kammer- und Speicherdruck sowie der Leitungsdruck auf Umgebungsdruck eingestellt haben. Dabei bleibt der Füllstand konstant, während die Luftmasse weiter leicht abnimmt (Schritt 3). Erst dann wird das Be- / Entlüftungsventil 24B wieder geschlossen (Schritt 4).

Daraufhin wird der Blutspeicher weiter mit Blut befüllt. Bei geöffnetem Bypassventil wird die Blutpumpe 6 solange betrieben, bis der Füllstand im Blutspeicher das Niveau des oberen Umschaltpunktes erreicht hat (Schritt 5). Dabei bleibt die Luftmasse im System konstant. Da der Kammer- und Speicherdruck sowie der Leitungsdruck mit den Druckgebern gemessen werden, kann der Füllstand in dem Blutspeicher fortlaufend berechnet werden. Die Steuer- und Recheneinheit berechnet den Füllstand in dem Blutspeicher und hält die Blutpumpe dann an, wenn der Füllstand das Niveau des oberen Umschaltpunktes erreicht hat. Dies wird später noch im Einzelnen beschrieben.

Nachdem die Steuer- und Recheneinheit die Blutpumpe 6 gestoppt hat, wird das Be- / Entlüftungsventil 24B wieder geöffnet, so dass sich der im System aufgebaute Druck auf den Umgebungsdruck entspannt (Schritt 6). Fig. 4 zeigt, dass der Füllstand konstant bleibt, während der Kammer- und Speicherdruck sowie der Leitungsdruck auf den Umgebungsdruck absinken. Da das Volumen, das von dem System eingeschlossen wird, d.h. das Volumen des Blutspeichers und des Luftspeichers sowie der Leitungen als auch die Drücke im System bekannt sind, kann die im System enthaltene Luftmasse berechnet werden. Dies wird später noch im Einzelnen beschrieben.

Als letzter Schritt der Initialisierung wird das Be- / Entlüftungsventil 24B geschlossen, wobei sich weder der Füllstand noch die Drücke sowie die Luftmasse im System verändern (Schritt 7). Das Be- / Entlüftungsventil bleibt während der gesamten Blutbehandlung geschlossen, wenn nicht eine erneute Initialisierung beispielsweise nach der Detektion einer Luftleckage erforderlich ist. Damit ist die Initialisierung abgeschlossen, und die Blutbehandlung startet mit der ersten venösen Phase.

In der ersten venösen Phase wird der Kompressor 20 bei geschlossenem Bypassventil 22 betrieben, wobei die venöse Schlauchklemme 12 geöffnet ist und die Blutpumpe 6 still steht. Während des Betriebs des Kompressors wird Luft aus dem Luftspeicher 18 komprimiert und dem Blutspeicher 11 zugeführt. Dadurch nehmen Kammer- und Leitungsdruck zu, während der Speicherdruck abnimmt. Gleichzeitig nimmt der Füllstand in dem Blutspeicher kontinuierlich ab, bis das Niveau des unteren Umschaltpunkts erreicht ist. Entscheidend ist, dass der Speicherdruck unterhalb des Kammer- und folglich auch des Leitungsdrucks liegt. Dabei wird zusätzlich angestrebt, dass der Speicherdruck unterhalb des Umgebungsdrucks liegt.

Daraufhin beginnt die arterielle Phase, in der der Blutspeicher wieder mit dem Patienten entnommenen Blut befüllt wird, woraufhin sich wieder die venöse Phase anschließt, in der das Blut aus dem Blutspeicher wieder dem Patienten zugeführt wird.

Der Luftspeicher ist so groß dimensioniert, dass auch am Ende der venösen Phase genügend Luft im System vorhanden ist, um den gewünschten Rückgabedruck in den Blutspeicher aufrechterhalten zu können. Um mit der gleichen Initialisierung sämtliche Betriebspunkte mit einem Rückgabedruck von 0 bis 500 mmHg relativ bei einem Schlagvolumen bis 60 ml einstellen zu können, wird in der Praxis ein Luftspeicher mit einem Speichervolumen von ca. 300 ml benötigt.

Der zeitliche Verlauf des Kammer- und Speicherdrucks sowie des Leitungsdrucks während der eigentlichen arteriellen und venösen Phasen nach der Initialisierung des Systems ist in Fig. 5 dargestellt, die einen Ausschnitt von Figur 4 zeigt.

Während der gesamten arteriellen Phase wird die Blutpumpe 6 betrieben, wobei der Kompressor 20 stillsteht. Die venöse Schlauchklemme 12 bleibt während der gesamten arteriellen Phase geschlossen.

Zu Beginn der arteriellen Phase öffnet die Steuer- und Recheneinheit 27 das Bypassventil 22, so dass die aus dem Blutspeicher 11 verdrängte Luft über die Bypassleitung 21 in den Luftspeicher 18 gelangt. Folglich steigt der Speicherdruck an, während der Kammer- und Leitungsdruck zunächst absinkt, um dann ebenfalls gleichsam mit dem Speicherdruck anzusteigen. Die in dem Blutspeicher und in dem zugehörigen Leitungsvolumen enthaltene Luftmasse nimmt damit kontinuierlich ab.

Sobald die in dem Blutspeicher und dem Leitungsvolumen enthaltene Luftmasse einen vorgegebenen Betrag erreicht hat, der sich aus dem gewünschten Schlagvolumen und dem gewünschten Rückgabedruck ergibt, schließt die Steuer- und Recheneinheit das Bypassventil. Folglich entstehen zwei getrennte Luftvolumina, d.h. das Luftvolumen in dem Blutspeicher mit den zugehörigen Leitungsabschnitten und das Volumen des Luftspeichers mit den zugehörigen Leitungsabschnitten. Daraufhin wird die Blutpumpe bei geschlossenem Bypassventil betrieben, so dass der Speicherdruck konstant bleibt, während die Luft in dem Blutspeicher und dem zugehörigen Leitungsvolumen so lange verdichtet wird, bis bei Erreichen des gewünschten Schlagvolumens auch der gewünschte Rückgabedruck erreicht ist.

Fig. 5 zeigt, dass zum Ende der arteriellen Phase der Kammer- und Leitungsdruck auf den gewünschten Rückgabedruck angestiegen sind, wobei der Speicherdruck während der gesamten arteriellen Phase immer unterhalb des Kammer- und Leitungsdrucks, insbesondere unterhalb des Umgebungsdrucks liegt. Dadurch ist ausgeschlossen, dass auch bei einem Störfall des Systems, beispielsweise bei einer Leckage des Kompressors, Luft aus dem Luftspeicher in den Blutspeicher gelangt.

Anstelle der Unterteilung der arteriellen Phase in ein erstes und zweites Zeitintervall sind auch andere Ausführungsformen möglich. Eine alternative Ausführungsform sieht vor, anstelle des Bypassventils 22 ein druckgesteuertes Ventil einzusetzen, das bei Erreichen eines dem Rückgabedruck entsprechenden Grenzdrucks öffnet, so dass in einem gewissen Zeitbereich ein konstanter Druck im Blutspeicher herrscht, womit sich auch die erfindungsgemäße Vorteile erzielen lassen.

Anschließend schaltet die Steuer- und Recheneinheit auf die venöse Phase um, wobei das Bypassventil geschlossen bleibt, die Blutpumpe angehalten und der Kompressor in Betrieb gesetzt sowie die venöse Schlauchklemme geöffnet wird. Der Kompressor wird während der gesamten venösen Phase betrieben, wobei die Blutpumpe stillsteht. Während der venösen Phase bleibt die venöse Schlauchklemme geöffnet und das Bypassventil geschlossen.

Der Kompressor fördert Luft aus dem Luftspeicher in den Blutspeicher, um einen Überdruck aufzubauen, so dass Blut aus dem Blutspeicher gefördert wird. Der Kompressor wird dabei derart betrieben, dass sich in dem Blutspeicher der gewünschte Rückgabedruck einstellt. Da dem Blutspeicher fortwährend Luft aus dem Luftspeicher zugeführt wird, nimmt der Speicherdruck kontinuierlich ab. Entscheidend ist wieder, dass der Speicherdruck immer unterhalb des Kammer- und Leitungsdrurks, insbesonere unterhalb des Umgebungsdruck liegt, so dass eine Luftinfusion aus dem Luftspeicher in den Patienten bei einem Störfall durch Entspannen des Volumens im Luftspeicher ausgeschlossen ist. Die venöse Phase ist dann beendet, wenn der Blutpegel in dem Blutspeicher wieder auf das Niveau des unteren Umschaltpunkts abgesunken ist. Dann schließt sich die nächste arterielle Phase an.

Nachfolgend wird die Berechnung des im Blutspeicher befindlichen Blutvolumens $V_{Blut}$ beschrieben, die von der Steuer- und Recheneinheit während des Betriebs der Dialysevorrichtung kontinuierlich oder in vorgegebenen Intervallen vorgenommen wird. Wenn das Blutvolumen bekannt ist, kann dies zum Vergleich mit den Umschaltpunkten von der arteriellen auf die venöse Phase oder umgekehrt verwendet werden.

Zur Berechnung des in dem Blutspeicher befindlichen zu fördernden Blutvolumens $V_{Blut}$ ist es zunächst notwendig, die Luftmasse im Blutspeicher zu bestimmen. Nach erfolgtem Druckausgleich gilt:

$$pV = \frac{m}{M_m} RT$$

mit:

p = Druck (absolut), V = Volumen, m = Masse, $M_m$ = molare Masse,
R = allg. Gaskonstante und T = Temperatur

Da $M_m$ und $R$ konstant sind und für die Luftmasse keine absoluten Werte benötigt werden, brauchen diese nicht explizit berücksichtigt zu werden. Bestimmt werden muss demnach nur

$$\frac{pV}{T} \equiv m.$$

Zur Bestimmung der gesamten Luftmasse muss die Summe aller Teilluftmassen, d.h. der Luftmasse im Blutspeicher, den zugehörigen Leitungen und im Luftspeicher gebildet werden. Hierzu müssen alle mit Luft gefüllten Volumina mit dem jeweils herrschenden Druck multipliziert und durch die Temperatur dividiert werden.

$$\left(\frac{pV}{T}\right)_{ges} = \frac{p_{Blutspeicher} \cdot V_{Blutspeicher/Luft}}{T_{Blutspeicher}} + \frac{p_{Leitung} \cdot V_{Leitung}}{T_{Leitung}} + \frac{p_{Luftspeicher} \cdot V_{Luftspeicher}}{T_{Luftspeicher}}$$

$$T_{Blutspeicher} \approx 273{,}15\ K + 36K$$

$$T_{Leitung} \approx \frac{T_{Blutspeicher} + T_{Luftspeicher}}{2}$$

Das Luftvolumen des Blutspeichers $V_{Blutspeicher/Luft}$ ändert sich während des Betriebs. Ist der Blutpegel auf der Höhe H des von dem Füllstandgeber 15 detektierten Niveaus, so entspricht

$$V_{Blutspeicher/Luft} = V_{Blutspeicher/Luft\,UT} - \Delta V_{Blutspeicher/\,UT - H},$$

wobei $V_{Blutspeicher/Luft\,UT}$ das Luftvolumen im Blutspeicher am unteren Umschaltpunkt UT und $\Delta V_{Blutspeicher/\,UT-H}$ die Volumendifferenz zwischen dem unteren Umschaltpunkt UT und der Höhe des von dem Füllstandgeber 15 detektierten Füllstands im Blutspeicher ist.

Am Ende der Initialisierung am oberen Umschaltpunkt OT befindet sich zusätzlich Blut mit dem gesamten Schlagvolumen $V_{Schlag}$ in dem Blutspeicher, so dass

$$V_{Blutspeicher/Luft} = V_{Blutspeicher/Luft\,UT} - V_{Schlag}$$

ist.

Die restlichen Volumina bleiben konstant. Dabei sind die Temperaturen in guter Näherung als konstant zu setzen, wenn keine Temperaturkompensation erfolgt. Vorzugsweise ist aber ein Temperatursensor zur Temperaturmessung zumindest im Luftspeicher vorgesehen, so dass eine Temperaturkompensation vorgenommen werden kann. Es können aber auch Temperatursensoren für die anderen Druckwerte vorgesehen sein.

Das geförderte Blutvolumen $V_{Blut}$ im Blutspeicher wird während des gesamten Zyklus bei jeder Programmschleife berechnet. Dabei wird anhand der gemessenen Drücke das im Blutspeicher eingeschlossene Luftvolumen $V_{Blutspeicher/Luft}$ berechnet und die Differenz von dem Luftvolumen im Blutspeicher bei dem unteren Umschaltpunkt UT und dem Luftvolumen des Blutspeichers gebildet.

$$V_{Blutspeicher/Luft} = \frac{\left(\dfrac{pV}{T}\right)_{ges} - \dfrac{P_{Leitung}V_{Leitung}}{T_{Leitung}} - \dfrac{P_{Luftspeicher}V_{Luftspeicher}}{T_{Luftspeicher}}}{\dfrac{P_{Blutspeicher}}{T_{Blutspeicher}}}$$

$$V_{Blut} = V_{Blutspeicher/Luft\,UT} - V_{Blutspeicher/Luft}$$

Die Gesamtluftmasse $\left(\dfrac{pV}{T}\right)_{ges}$ bleibt nach der Initialisierung des Systems unverändert, weil das Entlüftungsventil geschlossen bleibt. Da die Berechnung des Blutvolumens $V_{Blut}$ bei laufender Blutpumpe bzw. laufendem Kompressor erfolgt, wird eine Glättung der Drucksignale und des berechneten Blutvolumens $V_{Blut}$ durchgeführt.

Die Umschaltung von arterieller auf venöse Phase (OT) erfolgt, wenn die Differenz von dem berechneten Blutvolumen $V_{Blut}$ und dem eingestellten Schlagvolumen $V_{Schlag}$ gleich Null ist, und die Umschaltung von venöser auf arterielle Phase erfolgt, wenn das berechnete Blutvolumen gleich Null ist.

Im Folgenden wird beschrieben, wie die Steuer- und Recheneinheit den Zeitpunkt berechnet, zu dem innerhalb der arteriellen Phase zwischen dem ersten und zweiten Zeitintervall der arteriellen Phase umgeschaltet wird.

Wie bereits oben beschrieben, ist der Umschaltpunkt von arterieller zu venöser Phase und zurück durch einen einfachen Vergleich des Blutvolumens mit dem Schlagvolumen bzw. Null möglich. Die arterielle Phase teilt sich, wie bereits erwähnt, in ein erstes und ein zweites Zeitintervall auf. In der ersten arteriellen Phase fördert die Blutpumpe Blut über den Dialysator bei geöffnetem Bypassventil in den Blutspeicher. Dadurch wird der Unterdruck, der sich in der venösen Phase zuvor im Luftspeicher aufgebaut hat, zur Unterstützung der Pumpe genutzt. In der zweiten arteriellen Phase wird der Luftspeicher durch Schließen des Bypassventils von dem übrigen System abgekoppelt, und der Druck in dem Blutspeicher steigt durch das geförderte Blutvolumen stark an. Am Ende der zweiten arteriellen Phase soll im Blutspeicher der gewünschte Rückgabedruck oder Solldruck anstehen. Der Umschaltpunkt zwischen erster und zweiter arterieller Phase muss daher so gewählt werden, dass das noch ausstehende Blutvolumen bis zum oberen Umschaltpunkt OT in dem Blutspeicher und der Leitung den Solldruck aufbaut.

Berechnet werden muss demnach die Luftmasse, die bei Kompression auf das bei dem oberen Umschaltpunkt OT in dem Blutspeicher und der Leitung verfügbare Luftvolumen den Solldruck $p_{soll}$ aufbaut. Die Luftmasse in Blutspeicher und Leitung ist

$$\frac{p_{Blutspeicher}V_{Blutspeicher}}{T_{Blutspeicher}} + \frac{p_{Leitung}V_{Leitung}}{T_{Leitung}} = \left(\frac{pV}{T}\right)_{ges} - \frac{p_{Luftspeicher}V_{Luftspeicher}}{T_{Luftspeicher}} .$$

Beim Umschaltpunkt von dem ersten auf das zweite Zeitintervall der arteriellen Phase muss die in Blutspeicher und Leitung befindliche Luftmasse gleich der Luftmasse sein, die sich im oberen Umschaltpunkt OT in dem Blutspeicher und der Leitung befindet.

$$\left(\frac{pV}{T}\right)_{ges} - \frac{p_{Luftspeicher}V_{Luftspeicher}}{T_{Luftspeicher}} = \frac{p_{Soll} \cdot V_{Blutspeicher / LuftOT}}{T_{Blutspeicher}} + \frac{p_{Soll} \cdot V_{Leitung}}{T_{Leitung}}$$

Dabei ist $V_{Blutspeicher/LuftOT}$ das Luftvolumen im Blutspeicher am oberen Umschaltpunkt OT.

Die Steuer- und Recheneinheit überprüft während der arteriellen Phase, ob die obige Gleichung erfüllt ist. Sobald die Gleichung erfüllt ist, beginnt das zweite Zeitintervall der arteriellen Phase, wobei das Bypassventil geschlossen wird. Die Blutpumpe wird von der Steuer- und Recheneinheit in dem zweiten Zeitintervall mit der gleichen Förderrate betrieben, bis das gewünschte Schlagvolumen und somit der obere Umschaltpunkt OT erreicht ist.

Eine Leckage in dem abgeschlossenen Volumen kann eine Veränderung der im System eingeschlossenen Gasmenge zur Folge haben. Wenn das Leck im Überdruckbereich des Systems liegt, d.h. in dem Bereich des Blutspeichers oder der angrenzenden Leitungsabschnitte, so führt das Leck zu Verringerung der eingeschlossenen Luftmenge, wodurch der Füllstand des Bluts in dem Blutspeicher ansteigt. Dabei besteht die Gefahr, dass der Blutspeicher vollläuft und der Blutpegel bis zu der Hydrophobmembran ansteigt, so dass die arterielle Phase nicht mehr ordnungsgemäß beendet werden kann. Umgekehrt wird bei einer Leckage im Unterdruckbereich, d.h. im Gasspeicher oder durch Lufteintrag aus dem Blutsystem die eingeschlossene Luftmenge vergrößert, so dass der Füllstand des Bluts im Blutspeicher sinkt. Das kann dazuführen, dass der Blutpegel in der venösen Phase zu schnell abfällt, was zu unerwünschter Schaumbildung, im Extremfall sogar zum Luftalarm führen kann. Dies kann aber dadurch überwacht werden, dass die im System eingeschlossene Luftmenge beobachtet. Eine derartige Überwachung der Luftmenge kann bei der erfindungsgemäßen Vorrichtung vorgesehen sein.

Die Überwachung der Luftmenge zur Erkennung einer Leckage erfolgt dadurch, dass der Zeitpunkt erfasst wird, zu dem der Füllstandgeber 15 im Blutspeicher 11 den Füllstand detektiert, d.h. das Blut den vorgegebenen Pegel erreicht. Damit ist für diesen Zeitpunkt der tatsächliche Füllstand des Bluts bekannt. Dieser Wert wird mit dem zu diesem Zeitpunkt mit dem aus den Druckwerten berechneten Füllstand verglichen. Wenn die Differenz von dem gemessenen und berechneten Füllstand größer als ein vorgegebener Grenzwert ist, hat sich die in dem System eingeschlossene Luftmenge signifikant verändert, was auf eine Leckage im System zurückgeführt werden kann. In diesem Fall wird das System neu initialisiert. Tritt der Fehlerfall zu häufig auf, bricht die zentrale Steuer- und Recheneinheit 27 die Behandlung ab.

Eine alternative Auswertung sieht vor, nicht auf den gemessenen und berechneten Füllstand abzustellen, sondern den Zeitpunkt, zu dem der Füllstandgeber 15 den vorgegebenen Füllstand detektiert, mit dem berechneten Zeitpunkt zu vergleichen, zu dem sich der vorgegebene Füllstand einstellen sollte. Liegt eine signifikante Zeitdifferenz vor, wird auf eine Leckage im System geschlossen.

Eine weitere Ausführungsform sieht zusätzlich vor, eine Leckage im System in dem dem Blutspeicher abgewandten Teil hinter dem Filter 23 nach dem Ansteigen des Füllstands über das vom Füllstandgeber detektierte Niveau in der arteriellen Phase dadurch zu erkennen, dass mit dem Drucksensors 16 in dem Blutspeicher 11 ein zu stark ansteigender Druck erkannt wird, wobei der Druck und/oder der Druckanstieg pro Zeiteinheit einen vorgegebenen Grenzwert überschreitet. In diesem Fall führt das den Filter erreichende Blut zu einem Druckanstieg, was die Compliance in diesem Teil des Systems drastisch verringert. Dies trifft insbesondere für den Fall zu, in dem ein Drucksensor verwendet wird, der den Druck in direktem Kontakt, d.h. ohne kompressiblen Zwischenraum misst.

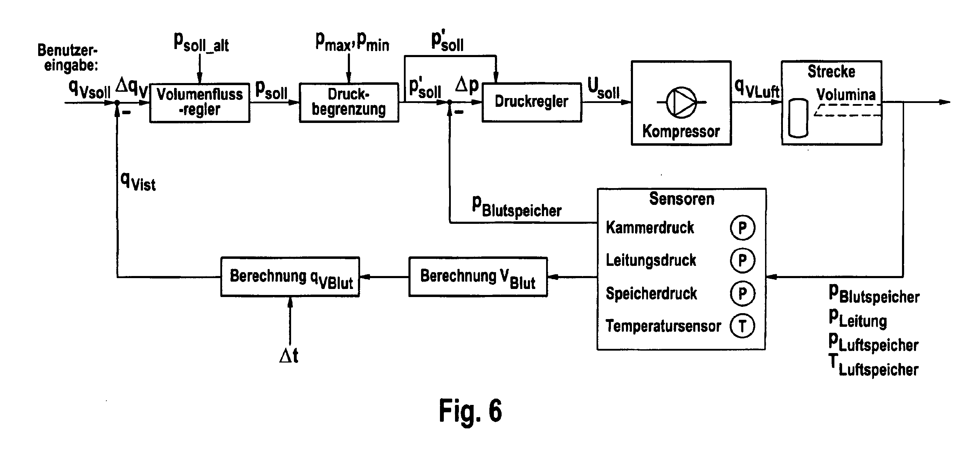

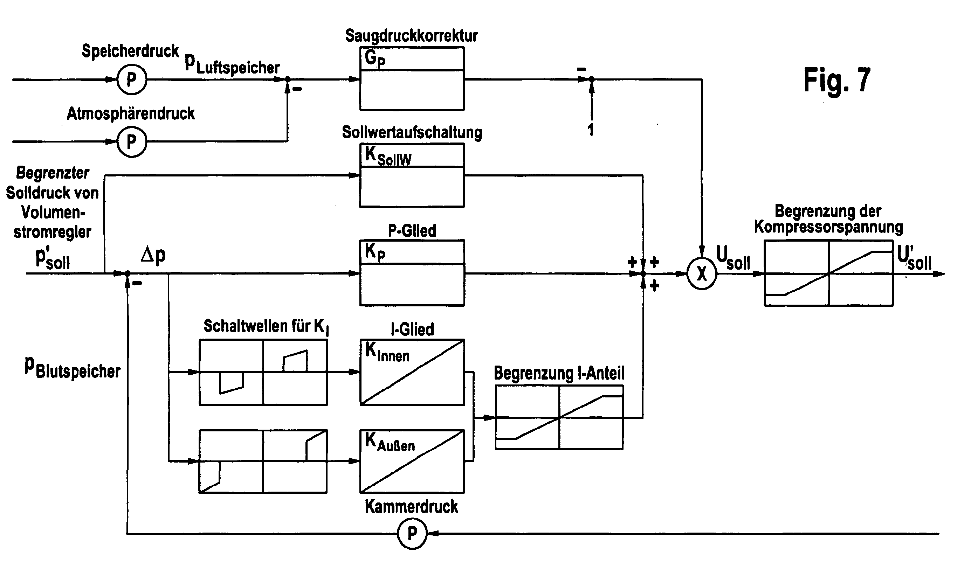

Nachfolgend wird die erfindungsgemäße Steuerungseinrichtung für die Dialysevorrichtung unter Bezugnahme auf die Figuren 6 und 7 im Einzelnen beschrieben, die Bestandteil der unter Bezugnahme auf die Figuren 3 bis 5 beschriebenen Dialysevorrichtung ist. Dabei macht die Steuerungseinrichtung von den Komponenten Gebrauch, die bereits in einer Dialysevorrichtung vorhanden sind. Die Steuerungseinrichtung ist Teil der zentralen Rechen- und Steuereinheit 27 der Dialysevorrichtung. In der zentralen Rechen- und Steuereinheit ist die in den Blockdiagrammen von den Figuren 6 und 7 gezeigte Reglerstruktur implementiert, deren Aufbau und Funktionsweise wie folgt ist.

Die Reglerstruktur sieht sowohl eine Regelung des Volumenstroms als auch des Drucks in der venösen Blutleitung 9 vor. Zu Beginn der regelmäßigen venösen Phase, d.h. nicht unmittelbar nach der Initialisierung des Systems (Fig. 3), ist der Blutspeicher 11 bereits mit Druck beaufschlagt. Der Blutspeicher 11 ist mit dem Solldruck zum Ende der letzten arteriellen Phase vorgespannt. Dadurch wird erreicht, dass sofort nach Öffnen der venösen Schlauchklemme 12 das Blut aus dem Blutspeicher 11 dem Patienten zurückgeführt werden kann.

Die Regelung erfolgt in einzelnen Takten mit einer vorgegebenen Schleifendauer, wobei typische Werte für die Schleifendauer beispielsweise 50 msec sind. Zunächst wird der vom Benutzer vorgegebene Sollwert für den Volumenstrom $qv_{soll}$ mit dem tatsächlichen Volumenstrom $q_{vist}$ zu Beginn der venösen Phase verglichen. Hierzu wird der tatsächliche Ist-Volumenstrom $q_{Vist}$ berechnet. Dabei gilt:

$$\Delta q_V = q_{V_{soll}} - q_{V_{ist}}$$

Die Regelung beruht darauf, dass mit einem äußeren Regler der Druck in dem Blutspeicher 11 ermittelt wird, bei dem sich der vorgegebene Volumenstrom in der venösen Blutleitung 9 einstellt, wobei mit einem inneren Regler die Mittel zum Verdichten von Gas 20, beispielsweise der Kompressor, derart betrieben werden, dass sich der ermittelte Soll-Druck $p_{soll}$ in der Blutkammer 11 auch einstellt.

Bei den mit dem inneren und äußeren Regler einzustellenden Solldrücken bzw. Grenzdrücken handelt es sich vorzugsweise um relative Drücke $\Delta p$ gegenüber dem Umgebungsdruck. Dabei wird vorzugsweise eine Verschiebung des Nullpunktes der relativen Grenz- oder Solldrücke aufgrund von Veränderungen des Umgebungsdrucks erfasst. Hierzu ist bei einer bevorzugten Ausführungsform ein Drucksensor vorgesehen, der den Umgebungsdruck misst.

Der Ist-Volumenstrom $q_{Vist}$ wird aus dem Quotienten des im Vergleich zur vorausgegangenen Programmschleife geänderten Blutvolumens $\Delta V_{Blut}$ und der Schleifendauer $\Delta t$ bestimmt, wobei das Blutvolumen für eine Schleife aus dem jeweiligen Kammerdruck, Leitungsdruck und Speicherdruck gegebenenfalls mit einer Temperaturkompensation berechnet wird, wie oben beschrieben ist.

Der Volumenflussregler muss auf jeden Fall langsamer sein, als der Druckregler zum Einregeln des Drucks, da ansonsten der Soll-Druck in dem Blutspeicher 11 zu schnell variiert wird. Bei dem Volumenstromregler handelt es sich vorzugsweise um einen I-Regler.

Der Algorithmus nutzt den Soll-Druck des letzten Schleifendurchlaufs und addiert die mit einem Faktor $K_{qv}$ multiplizierte Regeldifferenz $\Delta q_v$ hinzu.

$$p_{soll} = p_{Soll\_alt} + K_{qV} \cdot \Delta q_V$$

Bei der ersten venösen Phase nach der Initialisierung des Systems wird der Faktor $K_{qv}$ kleiner gewählt und der erste Soll-Druck $p_{Soll\_alt}$ mit $P_{atmos}$ + 50 hPa gewählt. Hierdurch wird gewährleistet, dass der Blutfluss langsam auf den gewünschten Blutfluss erhöht wird, so dass es nicht zu Druckspitzen kommen kann. Auch der Regelalgorithmus für den Druck wird im ersten Schleifendurchlauf langsamer gewählt.

Im Anschluss an die Ermittlung des Soll-Drucks $p_{Soll}$ wird der ermittelte Soll-Druck mit einer Druckbegrenzung begrenzt. Die Druckbegrenzung stellt zum einen sicher, dass der einzuregelnde Druck nicht größer ist als der vorgegebene maximal tolerierbare Rückgabedruck $p_{max}$ ist, d.h. der einzuregelnde Druck nicht über die physiologischen Grenzen hinaus ansteigt. Zum anderen stellt die Druckbegrenzung sicher, dass der einzuregelnde Druck nicht unter einen Mindestdruck, hier den Atmosphärendruck $p_{min}$ sinkt. Der obere Grenzwert für den Druck $p_{max}$ liegt über dem Grenzwert, bei dem die bei den bekannten Dialysevorrichtungen vorhandenen Alarmeinrichtungen einen Alarm auslösen, so dass sichergestellt werden kann, dass beispielsweise beim Verschluss der Patientenkonnektionsstelle ein Druckalarm ausgelöst wird, nicht aber nur der Zyklus angehalten wird, ohne einen Alarm auszulösen.

Der vom Volumenflussregler berechnete und begrenzte Soll-Druck wird im Anschluss daran vom Druckregler eingeregelt. Beim Druckregler handelt es sich vorzugsweise um einen PI-Regler mit geschaltetem Integrierer. Fig. 7 zeigt das Blockdiagramm des Druckreglers, wobei Fig. 7 als ein Ausschnitt von Fig. 6 zu verstehen ist. Der Druckregler erzeugt eine Steuerspannung $U_{soll}$ bzw. $U'_{soll}$, mit der der Kompressor 20 betrieben wird, wobei sich die Drehzahl (Leistung) des Kompressors mit steigender Steuerspannung erhöht.

Der Druckregler sieht eine Saugdruckkorrektur vor, bei der in Abhängigkeit von der Differenz zwischen dem Druck in dem Luftspeicher 18 und dem Atmosphärendruck, d.h. dem relativen Druck, das Ausgangssignal des Reglers erhöht wird. Da der Kompressor 20 im Luftspeicher 18 einen Unterdruck erzeugt, muss der Kompressor während der venösen Phase zunehmend gegen die entstehende Druckdifferenz arbeiten, so dass bei gleicher Drehzahl weniger Luft gefördert wird. Mit der Saugdruckkorrektur wird sichergestellt, dass die Steuerspannung entsprechend erhöht wird, um den Saugdruckabfall bei zunehmendem Unterdruck in dem Luftspeicher zu kompensieren.

Der Integrierer des Druckreglers verfügt über zwei Schaltschwellen ($\Delta p_{innen}$ und $\Delta p_{außen}$). Bei zu großen Sollwertabweichungen (äußere Schwelle) wird der I-Anteil zurückgenommen, um Überschwingungen zu vermeiden. Bei aber nur noch einer sehr geringen Regelabweichung (innere Schwelle) kann der I-Anteil zu Null gesetzt werden. Der I-Anteil berechnet sich unter Berücksichtigung relativer Werte für den Druck bezogen auf den Umgebungsdruck wie folgt:

$$I_{neu} = I_{alt} + \Delta p \cdot K_I$$

$$K_I = K_{I\_außen} \text{ falls } \Delta p > \Delta p_{außen}$$

$$K_I := K_{I\_innen} \text{ falls } \Delta p_{innen} < \Delta p < \Delta p_{außen}$$

$$K_I = 0 \qquad \text{ falls } \Delta p < \Delta p_{innen}$$

Der maximale I-Anteil wird begrenzt, um einer zu großen Regelabweichung vorzubeugen.
Der Reglerausgang berechnet sich als Summe des P- und I-Anteils mit der Sollwertaufschaltung:

$$S_{Regler} = \Delta p \cdot K_p + I_{neu} + p'_{Soll} \cdot K_{SollW}$$

Der Reglerausgang wird anschließend, wie bereits oben beschrieben, durch die Saugdruckkorrektur in Abhängigkeit von dem absoluten Druck im Luftspeicher 18 unterstützt:

$$S_{Ausgang} = S_{Regler} \cdot (1 - (G_p \cdot (p_{Luftspeicher} - p_{atmos})))$$

Da der Kompressor 20 nur mit einer maximalen Betriebsspannung betrieben werden kann, wird die Steuerspannung $U_{soll}$ auf die maximale und minimale Steuerspannung begrenzt. Der Kompressor wird mit der Steuerspannung $U'_{Soll}$ betrieben.

Zu beachten ist, dass sowohl der Soll-Druck als auch der Integrator des Druckreglers aus der vorangegangenen Programmschleife bzw. den vorangegangenen Zyklen benötigt wird. Außerdem wird der Soll-Druck zur Bestimmung des Umschaltpunkts von dem ersten auf das zweite Zeitintervall der arteriellen Phase benötigt. Daher werden diese beiden Werte zwischengespeichert.

**Patentansprüche**

1. Vorrichtung zur Steuerung einer Einrichtung zum Fördern von Blut in einer Blutleitung eines extrakorporalen Blutkreislaufs einer extrakorporalen Blutbehandlungsvorrichtung mit
   Mitteln (28; 62) zum Vorgeben eines bestimmten Volumenstroms für das in der Blutleitung strömende Blut,
   Mitteln (27; 61) zum Erzeugen eines Steuersignals für die Einrichtung zum Fördern von Blut in der Blutleitung, die derart ausgebildet sind, dass ein Steuersignal für die Einrichtung zum Fördern von Blut erzeugt wird, das derart bemessen ist, dass das Blut in der Blutleitung mit dem vorgegebenen Volumenstrom gefördert wird,
   Mitteln (28; 62) zum Vorgeben eines bestimmten ersten Grenzwertes für den Druck in der Blutleitung,
   Mitteln (27; 64, 65) zum Bestimmen des Drucks in der Blutleitung und
   Mitteln (27, 61) zum Vergleichen des Drucks in der Blutleitung mit dem vorgegebenen ersten Grenzwert für den Druck in der Blutleitung,
   einer bei einem bestimmten zweiten Grenzwert für den Druck in der Blutleitung einen Alarm auslösenden Alanneinrichtung, wobei der erste Grenzwert über dem zweiten Grenzwert liegt,
   wobei die Mittel (27, 61) zum Erzeugen eines Steuersignals für die Einrichtung zum Fördern von Blut derart ausgebildet sind, dass bei Erreichen des vorgegebenen ersten Grenzwertes für den Druck in der Blutleitung ein Steuersignal für die Einrichtung zum Fördern von Blut erzeugt wird, das derart bemessen ist, dass sich beim Fördern des Bluts in der Blutleitung ein Druck einstellt, der dem ersten Grenzwert entspricht.

2. Extrakorporale Blutbehandlungsvorrichtung mit einer Einrichtung (11; 56) zum Fördern von Blut in einer Blutleitung (8, 54; 9, 54) eines extrakorporalen Blutkreislaufs, der eine zu einer Blutbehandlungseinheit (2; 50) führende arterielle Blutleitung (8; 54) und eine von der Blutbehandlungseinheit abgehende venöse Blutleitung (9; 54) aufweist, und einer Vorrichtung zur Steuerung der Einrichtung zum Fördern von Blut nach Anspruch 1.

3. Extrakorporale Blutbehandlunpsvorrichtung nach Anspruch 2, **dadurch gekennzeichnet**, **dass** die Blutbehandlungsvorrichtung als eine Blutbehandlungsvorrichtung für den Betrieb mit zwei Patientenkonnektionsstellen ausgebildet ist, wobei die arterielle Blutleitung (54) eine arterielle Patientenkonnektionsstelle (54a) und die venöse Blutleitung (55) eine venöse Patientenkonnektionsstelle (55a) aufweist.

4. Extrakorporale Blutbehandlungsvorrichtung nach Anspruch 3, **dadurch gekennzeichnet, dass** die Einrichtung zum Fördern von Blut als eine Blutpumpe (56) ausgebildet ist, die in der arteriellen Blutleitung (54) angeordnet ist.

5. Extrakorporale Blutbehandlungsvorrichtung nach Anspruch 4, dadurch **gekennzeichnet, dass** die Mittel zum Bestimmen des Drucks als Mittel zum Bestimmen des Drucks in der Blutleitung (55) stromab der Blutpumpe (56) ausgebildet sind.

6. Extrakorporale Blutbehandlungsvorrichtung nach Anspruch 4, **dadurch gekennzeichnet**, **dass** die Mittel zum Bestimmen des Drucks als Mittel zum Bestimmen des Drucks in der Blutleitung (54) stromauf der Blutpumpe (56) ausgebildet sind.

7. Extrakorporale Blutbehandlungsvorrichtung nach Anspruch 2, **dadurch gekennzeichnet,** dass die Blutbehandlungsvorrichtung als eine Blutbehandlungsvorrichtung für den Betrieb mit einer Patientenkonnektionsstelle ausgebildet ist, wobei die arterielle Blutleitung (8) und die venöse Blutleitung (9) eine gemeinsame Patientenkonnektionsstelle (10) aufweisen.

8. Extrakorporale Blutbehandlungsvorrichtung nach Anspruch 7, **dadurch gekennzeichnet**, **dass** die Blutbehandlungsvorrichtung in der venösen Blutleitung (9) des extrakorporalen Blutkreislaufs (1) angeordnete Mittel (11) zum Sammeln von Blut aufweist, die ein vorgegebenes Volumen umfassen, wobei die Einrichtung zum Fördern von Blut als eine Einrichtung (20) zur Erzeugung eines vorgegebenen Drucks in den Mitteln zum Sammeln von Blut ausgebildet ist, so dass in den Mitteln zum Sammeln von Blut gesammeltes Blut aus den Mitteln zum Sammeln von Blut verdrängt wird.

9. Extrakorporale Blutbehandlungsvorrichtung nach Anspruch 8, dadurch **gekennzeichnet, dass** die Einrichtung zum Fördern von Blut Mittel (18) zum Speichern von Luft aufweist, die ein abgeschlossenes Volumen umfassen, und Mittel (20) zum Verdichten von Luft aufweist, die derart mit den Mitteln zum Speichern von Luft und den Mitteln (11) zum Sammeln von Blut über einen Verbindungspfad (19) in Strömungsverbindung stehen, dass Luft aus den Mitteln zum Speichern von Luft unter Verdrängung des in den Mitteln zum Sammeln von Blut gesammelten Bluts in die Mittel zum Sammeln von Blut überführbar ist.

10. Extrakorporale Blutbehandlungsvorrichtung nach Anspruch 8 oder 9, **dadurch gekennzeichnet, dass** die Mittel (27) zur Erzeugung eines Steuersignals aufweisen:

einen ersten Regelkreis, der Mittel zum Berechnen eines Soll-Drucks für das abgeschlossene Volumen der Mittel (11) zum Sammeln von Blut aufweist, der derart bemessen ist, dass sich in der venösen Blutleitung (9) der vorgegebene Volumenstrom einstellt,
und einen zweiten Regelkreis zur Erzeugung eines Steuersignals für die Mittel (20) zum Verdichten von Luft, das derart bemessen ist, dass sich in dem abgeschlossenen Volumen der Mittel (11) zum Sammeln von Blut der berechnete Soll-Druck einstellt, bei dem sich der vorgegebene Volumenstrom in der venösen Blutleitung (9) einstellt.

11. Extrakorporale Blutbehandlungsvorrichtung nach Anspruch 10, **dadurch gekennzeichnet, dass** der erste Regelkreis Mittel zum Begrenzen des Soll-Drucks auf einen vorgegebenen Grenzwert aufweist.

12. Extrakorporale Blutbehandlungsvorrichtung nach Anspruch 10 oder 11, **dadurch** gekennzeichnet, **dass** der erste Regelkreis als I-Regler ausgebildet ist, der den Soll-Druck durch zeitliche Integration der Differenz von dem vorgegebenen Volumenstrom und dem Ist-Volumenstrom bestimmt.

13. Extrakorporale Blutbehandlungsvorrichtung nach einem der Ansprüche 10 bis 12, **dadurch gekennzeichnet, dass** der zweite Regelkreis als PI-Regler ausgebildet ist.

14. Extrakorporale Blutbehandlungsvorrichtung nach einem der Ansprüche 10 bis 13, **dadurch gekennzeichnet, dass** der zweite Regelkreis Mittel zur Kompensation aufweist, die derart ausgebildet sind, dass das Steuersignal für die Mittel (20) zum Verdichten von Luft in Abhängigkeit von dem Druck in dem abgeschlossenen Volumen der Mittel (18) zum Speichern von Luft verändert wird.

15. Extrakorporale Blutbehandlungsvorrichtung nach einem der Ansprüche 9 bis 14, **dadurch gekennzeichnet, dass** die Mittel (27) zur Erzeugung eines Steuersignals Mittel zur Ermittlung des in einem abgeschlossenen Volumen eingeschlossenen Blutvolumens und Mittel zum Berechnen der Abnahme des eingeschlossenen Blutvolumens in einem vorgegebenen Zeitintervall aufweisen.

16. Extrakorporale Blutbehandlungsvorrichtung nach Anspruch 15, **dadurch gekennzeichnet, dass** die Mittel zur Ermittlung des Blutvolumens aufweisen:

Mittel zum Messen des Drucks in dem abgeschlossenen Volumen der Mittel (11) zum Sammeln von Blut,
Mittel zum Messen des Drucks in dem abgeschlossenen Volumen des Verbindungspfads (19) zwischen den Mitteln (11) zum Sammeln von Blut und den Mitteln (20) zum Verdichten von Luft, und
Mittel zum Messen des Drucks in dem abgeschlossenen Volumen der Mittel (18) zum Speichern von Luft, wobei die Mittel zur Ermittlung des Blutvolumens derart ausgebildet sind, dass das Blutvolumen auf der Grundlage des gemessenen Drucks in dem abgeschlossenen Volumen der Mittel (11) zum Sammeln von Blut, dem abgeschlossenen Volumen des Verbindungspfads (19) zwischen den Mitteln zum Sammeln von Blut und den Mitteln zum Verdichten von Luft und dem abgeschlossenen Volumen der Mittel (18) zum Speichern von Luft berechnet wird.

## Claims

1. Apparatus for controlling a device for transporting blood in a blood conduit of an extracorporeal blood circuit of an extracorporeal blood treatment apparatus, comprising
means (28; 62) for setting a particular volume flow for the blood flowing in the blood conduit,
means (27; 61) for producing a control signal for the device for transporting blood in the blood conduit, which means are designed such that a control signal for the device for transporting blood is of such a size that the blood in the blood conduit is transported at the predetermined volume flow rate,
means (28; 62) for setting a particular first limit value for the pressure in the blood conduit,
means (27; 64, 65) for determining the pressure in the blood conduit, and
means (27; 61) for comparing the pressure in the blood conduit with the predetermined first limit value for the pressure in the blood conduit,
an alarm device which triggers an alarm at a particular second limit value for the pressure in the blood conduit, wherein the first limit value is above the second limit value,
wherein the means (27, 61) for producing a control signal for the device for transporting blood are configured such that a control signal for the device for transporting blood is produced when the predetermined first limit value for the pressure in the blood conduit is reached, which control signal is of such a size that when the blood is transported in the blood conduit, a pressure is produced that corresponds to the first limit value.

2. Extracorporeal blood treatment apparatus comprising a device (11; 56) for transporting blood in a blood conduit (8, 54; 9, 54) of an extracorporeal blood circuit that has an arterial blood conduit (8; 54) leading to a blood treatment unit (2; 50) and a venous blood conduit (9; 54) leading away from the blood treatment unit, and an apparatus for controlling the device for transporting blood according to claim 1.

3. Extracorporeal blood treatment apparatus according to claim 2, **characterised in that** the blood treatment apparatus is formed as a blood treatment apparatus for operation with two patient connection points, the arterial blood conduit (54) having an arterial patient connection point (54a) and the venous blood conduit (55) having a venous patient connection point (55a).

4. Extracorporeal blood treatment apparatus according to claim 3, **characterised in that** the device for transporting blood is formed as a blood pump (56) arranged in the arterial blood conduit (54).

5. Extracorporeal blood treatment apparatus according to claim 4, **characterised in that** the means for determining the pressure are formed as means for determining the pressure in the blood conduit (55) downstream of the blood pump (56).

6. Extracorporeal blood treatment apparatus according to claim 4, **characterised in that** the means for determining the pressure are formed as means for determining the pressure in the blood conduit (54) upstream of the blood pump (56).

7. Extracorporeal blood treatment apparatus according to claim 2, **characterised in that** the blood treatment apparatus is formed as a blood treatment apparatus for operation with one patient connection point, the arterial blood conduit (8) and the venous blood conduit (9) having a common patient connection point (10).

8. Extracorporeal blood treatment apparatus according to claim 7, **characterised in that** the blood treatment apparatus comprises means (11) for collecting blood which have a predetermined volume and are arranged in the venous blood conduit (9) of the extracorporeal blood circuit (1), the device for transporting blood being formed as a device (20) for producing a predetermined pressure in the means for collecting blood, such that blood that has collected in the means for collecting blood can be displaced out of the means for collecting blood.

9. Extracorporeal blood treatment apparatus according to claim 8, **characterised in that** the device for transporting blood comprises means (18) for storing air which have a closed volume, and means (20) for compressing air which are connected in terms of flow, via a connection path (19), to the means for storing air and the means (11) for collecting blood, such that air from the means for storing air can be conveyed into the means for collecting blood, thereby displacing the blood that has collected in the means for collecting blood.

10. Extracorporeal blood treatment apparatus according to either claim 8 or claim 9, **characterised in that** the means (27) for producing a control signal comprise:

a first control loop having means for calculating a setpoint pressure for the closed volume of the means (11) for collecting blood, which pressure is of such a size that the predetermined volume flow rate is produced in the venous blood conduit (9),
and a second control loop for generating a control signal for the means (20) for compressing air, which signal is of such a size that the calculated setpoint pressure, at which the predetermined volume flow rate is produced in the venous blood conduit (9), is produced in the closed volume of the means (11) for collecting blood.

11. Extracorporeal blood treatment apparatus according to claim 10, **characterised in that** the first control loop comprises means for limiting the setpoint pressure to a predetermined limit value.

12. Extracorporeal blood treatment apparatus according to either claim 10 or claim 11, **characterised in that** the first control loop is formed as an integral controller that determines the setpoint pressure by means of temporal integration of the difference between the predetermined volume flow and the actual volume flow.

13. Extracorporeal blood treatment apparatus according to any of claims 10 to 12, **characterised in that** the second control loop is formed as a PI controller.

14. Extracorporeal blood treatment apparatus according to any of claims 10 to 13, **characterised in that** the second control loop comprises compensating means that are configured such that the control signal for the means (20) for compressing air is altered on the basis of the pressure in the closed volume of the means (18) for storing air.

15. Extracorporeal blood treatment apparatus according to any of claims 9 to 14, **characterised in that** the means (27) for producing a control signal comprise means for determining the blood volume enclosed in a closed volume, and means for calculating the decrease in the enclosed blood volume in a predetermined time interval.

16. Extracorporeal blood treatment apparatus according to claim 15, **characterised in that** the means for determining the blood volume comprise:

16

means for measuring the pressure in the closed volume of the means (11) for collecting blood,
means for measuring the pressure in the closed volume of the connection path (19) between the means (11) for collecting blood and the means (20) for compressing air, and
means for measuring the pressure in the closed volume of the means (18) for storing air,
the means for determining the blood volume being configured such that the blood volume is calculated on the basis of the measured pressure in the closed volume of the means (11) for collecting blood, the closed volume of the connection path (19) between the means for collecting blood and the means for compressing air, and the closed volume of the means (18) for storing air.

## Revendications

1.  Dispositif permettant de commander un dispositif destiné à transporter le sang dans une conduite de sang d'un circuit de circulation extracorporelle du sang d'un dispositif de traitement extracorporel du sang, comportant
    des moyens (28 ; 62) pour prédéfinir un flux volumique déterminé pour le sang circulant dans la conduite de sang,
    des moyens (27 ; 61) pour générer un signal de commande pour le dispositif destiné à transporter le sang dans la conduite de sang, lesquels sont configurés de manière à générer un signal de commande pour le dispositif destiné à transporter le sang, lequel est déterminé de telle sorte que le sang est transporté dans la conduite de sang avec le flux volumique prédéfini,
    des moyens (28 ; 62) pour fixer une première valeur limite déterminée pour la pression dans la conduite de sang,
    des moyens (27 ; 64, 65) pour déterminer la pression dans la conduite de sang, et des moyens (27, 61) pour comparer la pression dans la conduite de sang à la première valeur limite fixée pour la pression dans la conduite de sang,
    un dispositif d'alarme, déclenchant une alarme en présence d'une deuxième valeur limite déterminée pour la pression dans la conduite de sang, la première valeur limite étant supérieure à la deuxième valeur limite,
    lesdits moyens (27, 61) destinés à générer un signal de commande pour le dispositif destiné à transporter le sang étant configurés de telle sorte que lorsque la première valeur limite fixée pour la pression dans la conduite de sang est atteinte, un signal de commande pour le dispositif destiné à transporter le sang est généré, lequel est déterminé de manière à établir une pression correspondant à la première valeur limite, lorsque le sang est transporté dans la conduite de sang.

2.  Dispositif de traitement extracorporel du sang, comportant un dispositif (11 ; 56) destiné à transporter le sang dans une conduite de sang (8, 54 ; 9, 54) d'un circuit de circulation extracorporelle du sang, qui comporte une conduite artérielle de sang (8 ; 54) menant vers une unité de traitement du sang (2 ; 50) et une conduite veineuse de sang (9 ; 54) partant de ladite unité de traitement du sang, et un dispositif pour commander le dispositif destiné à transporter le sang selon la revendication 1.

3.  Dispositif de traitement extracorporel du sang selon la revendication 2, **caractérisé en ce que** le dispositif de traitement du sang est réalisé sous la forme d'un dispositif de traitement du sang pour un mode de fonctionnement avec deux points de connexion au patient, la conduite artérielle de sang (54) comportant un abord artériel au patient (54a) et la conduite veineuse de sang (55) comportant un abord veineux au patient (55a).

4.  Dispositif de traitement extracorporel du sang selon la revendication 3, **caractérisé en ce que** le dispositif destiné à transporter le sang est réalisé sous la forme d'une pompe à sang (56) qui est agencée dans la conduite artérielle de sang (54).

5.  Dispositif de traitement extracorporel du sang selon la revendication 4, **caractérisé en ce que** les moyens pour déterminer la pression sont réalisés sous la forme de moyens destinés à déterminer la pression dans la conduite de sang (55) en aval de la pompe à sang (56).

6.  Dispositif de traitement extracorporel du sang selon la revendication 4, **caractérisé en ce que** les moyens pour déterminer la pression sont réalisés sous la forme de moyens destinés à déterminer la pression dans la conduite de sang (54) en amont de la pompe à sang (56).

7.  Dispositif de traitement extracorporel du sang selon la revendication 2, **caractérisé en ce que** le dispositif de traitement du sang est réalisé sous la forme d'un dispositif de traitement du sang pour un mode de fonctionnement avec un point de connexion au patient, la conduite artérielle de sang (8) et la conduite veineuse de sang (9) comportant un abord au patient (10) commun.

**8.** Dispositif de traitement extracorporel du sang selon la revendication 7, **caractérisé en ce que** le dispositif de traitement du sang comporte des moyens (11) pour collecter le sang, lesquels sont agencés dans la conduite veineuse de sang (9) du circuit de circulation extracorporelle du sang (1) et lesquels comportent un volume prédéfini, le dispositif destiné à transporter le sang étant réalisé sous la forme d'un dispositif (20) pour produire une pression prédéfinie dans les moyens pour collecter le sang, de telle sorte que le sang contenu dans les moyens pour collecter le sang sont poussés hors des moyens pour collecter le sang.

**9.** Dispositif de traitement extracorporel du sang selon la revendication 8, **caractérisé en ce que** le dispositif destiné à transporter le sang comporte des moyens (18) pour stocker l'air qui comportent un volume fermé, et des moyens (20) pour comprimer l'air qui sont en liaison fluidique, via une voie de liaison (19), avec les moyens pour stocker l'air et les moyens (11) pour collecter le sang, de telle sorte que l'air peut être transporté hors des moyens pour stocker l'air vers les moyens pour collecter le sang sous l'effet du refoulement du sang contenu dans les moyens pour collecter le sang.

**10.** Dispositif de traitement extracorporel du sang selon la revendication 8 ou 9, **caractérisé en ce que** les moyens (27) pour générer un signal de commande comportent :

un premier circuit de réglage qui comporte des moyens pour calculer une pression de consigne pour le volume fermé des moyens (11) pour collecter le sang, lequel est déterminé de telle sorte que le flux volumique prédéfini s'établit dans la conduite veineuse de sang (9),
et un deuxième circuit de réglage pour générer un signal de commande pour les moyens (20) pour comprimer l'air, lequel est déterminé de telle sorte que dans le volume fermé des moyens (11) pour collecter le sang s'établit la pression de consigne calculée, à laquelle s'établit le flux volumique prédéfini dans la conduite veineuse de sang (9).

**11.** Dispositif de traitement extracorporel du sang selon la revendication 10, **caractérisé en ce que** le premier circuit de réglage comporte des moyens pour limiter la pression de consigne à une valeur limite prédéfinie.

**12.** Dispositif de traitement extracorporel du sang selon la revendication 10 ou 11, **caractérisé en ce que** le premier circuit de réglage est réalisé sous la forme d'un régulateur à action intégrale, qui détermine la pression de consigne par une intégration en fonction du temps de la différence entre le flux volumique prédéfini et le flux volumique réel.

**13.** Dispositif de traitement extracorporel du sang selon l'une quelconque des revendications 10 à 12, **caractérisé en ce que** le deuxième circuit de réglage est réalisé sous la forme d'un régulateur à action proportionnelle et intégrale.

**14.** Dispositif de traitement extracorporel du sang selon l'une quelconque des revendications 10 à 13, **caractérisé en ce que** le deuxième circuit de réglage comporte des moyens de compensation, qui sont configurés de telle sorte que le signal de commande pour les moyens (20) pour comprimer l'air est modifié en fonction de la pression dans le volume fermé des moyens (18) pour stocker l'air.

**15.** Dispositif de traitement extracorporel du sang selon l'une quelconque des revendications 9 à 14, **caractérisé en ce que** les moyens (27) pour générer un signal de commande comportent des moyens pour déterminer le volume de sang contenu dans un volume fermé et des moyens pour calculer la diminution du volume de sang contenu pendant un intervalle de temps prédéfini.

**16.** Dispositif de traitement extracorporel du sang selon la revendication 15, **caractérisé en ce que** les moyens pour déterminer le volume de sang comportent :

des moyens pour mesurer la pression dans le volume fermé des moyens (11) pour collecter le sang,
des moyens pour mesurer la pression dans le volume fermé de la voie de liaison (19) entre les moyens (11) pour collecter le sang et les moyens (20) pour comprimer l'air, et
des moyens pour mesurer la pression dans le volume fermé des moyens (18) pour stocker l'air,
lesdits moyens pour déterminer le volume de sang étant configurés de telle sorte que le volume de sang est calculé sur la base de la pression mesurée dans le volume fermé des moyens (11) pour collecter le sang, dans le volume fermé de la voie de liaison (19) entre les moyens pour collecter le sang et les moyens pour comprimer l'air, et dans le volume fermé des moyens (18) pour stocker l'air.

**Fig. 1**

EP 2 431 064 B1

Fig. 2

# Fig. 3

EP 2 431 064 B1

**Fig. 4**

| Initialisierung | | | | | | | Betrieb | | |
|---|---|---|---|---|---|---|---|---|---|
| 1 | 2 | 3 | 4 | 5 | 6 | 7 | 1. venöse Phase | arterielle Phase | venöse Phase |

**Füllstand**
oberer Umschaltpunkt
Leveldetektor
unterer Umschaltpunkt

Zeit

**Drücke**
gewünschter Rückgabedruck
Umgebungsdruck

Leitungsdruck
Kammerdruck
Speicherdruck

Zeit

**Gesamt-Luftmasse im System**

Zeit

EP 2 431 064 B1

Fig. 5

Fig. 6

Fig. 7

Begrenzung der Kompressorsorspannung

$U'_{soll}$

$U_{soll}$

Saugdruckkorrektur

$G_P$

Sollwertaufschaltung

$K_{SollW}$

P-Glied

$K_P$

I-Glied

$K_{Innen}$

$K_{Außen}$

Kammerdruck

Begrenzung I-Anteil

Schaltwellen für $K_I$

Speicherdruck $p_{Luftspeicher}$

Atmosphärendruck

Begrenzter Solldruck von Volumen-stromregler

$p'_{soll}$

$\Delta p$

$p_{Blutspeicher}$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0472480 A
- DE 102005001779 A1
- US 5178603 A
- US 20030152482 A1
- WO 03072942 A1